# EUROPEAN PATENT SPECIFICATION

(11) **EP 4 190 368 B1**
(45) Date of publication and mention of the grant of the patent: **24.06.2026**
(21) Application number: 21848791.6
(22) Date of filing: 30.07.2021
(51) Int. Cl.: A61L 31/04, A61L 31/12, A61L 31/14, A61L 31/16

(54) **COMPOSITION FOR PREVENTING ADHESION**
ZUSAMMENSETZUNG ZUR VERHINDERUNG VON ADHÄSION
COMPOSITION POUR EMPÊCHER LES ADHÉRENCES

(30) Priority: 31.07.2020 JP 2020130896
(43) Date of publication of application: 07.06.2023
(73) Proprietor: Mochida Pharmaceutical Co., Ltd., Tokyo 160-8515 (JP); The University of Tokyo, Bunkyo-ku, Tokyo 113-8654 (JP)
(72) Inventor: ITO Taichi, Tokyo 113-8654 (JP); OHTA Seiichi, Tokyo 113-8654 (JP); HASEGAWA Kiyoshi, Tokyo 113-8654 (JP); ISAJI Mitsuko, Tokyo 160-8515 (JP); TANAKA Daichi, Tokyo 160-8515 (JP)
(74) Representative: dompatent
(86) International application number: PCT/JP2021/028275
(87) International publication number: WO 2022/025229

(56) References cited:
- EP-A1- 3 485 921
- WO-A1-2018/012605
- WO-A1-2019/138583
- JP-A- 2016 502 874
- US-A1- 2012 039 959
- EVA ESSER ET AL: "Preparation of well-defined calcium cross-linked alginate films for the prevention of surgical adhesions", JOURNAL OF BIOMEDICAL MATERIALS RESEARCH PART B: APPLIED BIOMATERIALS, vol. 101B, no. 5, 24 April 2013 (2013-04-24), US, pages 826 - 839, XP055662813, ISSN: 1552-4973, DOI: 10.1002/jbm.b.32886

## Description

### [Technical Field]

The present invention relates to a material for preventing adhesion and a production method thereof.

### [Background Art]

Adhesions refer to a state where surfaces of tissues that should be separated from each other are connected or fused to each other via a fibrous tissue. Adhesions occur in association with injury or inflammation upon which an exudate containing fibrin is emitted on the surface of the tissue, where this exudate is organized such that the tissue surfaces are connected or fused. Adhesions are caused by an injury generated on a surface of a tissue upon a surgical operation, inflammation caused by an injury, and inflammation caused by drying of a tissue surface upon a surgical operation.

Adhesions sometimes cause infertility, bowel passing disorder and chronic pelvic pain. Moreover, in order to separate adhesions that were caused after a surgical operation, another surgical operation may be required. For example, while multiple times of operations may be effective for a recurrent case of liver cancer, judgement of the propriety of a repeated surgery, risks of the treatment, an amount of bleeding upon the operation, operation time and the like are all largely dependent on the prevention of adhesions following the previous operation. Accordingly, there is a need for preventing adhesions and various means have been adopted to date for preventing adhesions.

Some of such means for preventing adhesions involve providing a physical barrier between an injury or an inflammation site and the adjacent tissue to prevent the tissues from connecting or fusing with each other. A sheet-like barrier is known as such a physical barrier.

Specifically, examples of such a sheet-like barrier include a polytetrafluoroethylene (PTFE) film (Preclude (trade name) (WL Gore and Associates, Inc.)), a sheet containing hyaluronic acid (HA) and carboxymethyl cellulose (CMC) (Seprafilm (trade name) (Genzyme GmbH)), and an oxidized regenerated cellulose sheet (INTERCEED (trade name) (Johnson & Johnson)). Since the PTFE film from among these is not biodegradable, it has a problem of remaining in the body. On the other hand, the sheet containing HA and CMC as well as the oxidized regenerated cellulose sheet are biodegradable but they are unable to completely prevent serious adhesions such as an adhesion caused after hepatic resection, and thus they require further improvement to be effective in adhesion prevention.

Now, it is known to make a biocompatible material, selected from proteins such as collagen or polysaccharides such as carboxymethyl cellulose, hyaluronic acid or alginic acid, into a sheet or particles that can be used as a medical absorbing material, a medical patch, a material for preventing adhesion, a biological tissue reinforcement material or the like (Patent Literature 1 to 6).

In addition, Patent Literature 7 and 8 describe materials for preventing adhesion including a biocompatible sponge-like laminate that includes first and second sponge-like layers containing low-endotoxin monovalent metal salts of alginic acid which are at least partially crosslinked with a curing agent, with the weight-average molecular weight of the monovalent metal salt of alginic acid in the first layer being higher than the weight-average molecular weight of the monovalent metal salt of alginic acid in the second layer.

In addition, Patent Literature 9 describes a device for preventing an adhesion following a surgery, containing an alginic acid composition and a crosslinking agent.

### [Prior Art Documents]

### [Patent Literature]

[Patent Literature 1] Japanese Patent Application Publication No. S48-79870
[Patent Literature 2] Japanese Patent Application Publication No. 2003-126235
[Patent Literature 3] WO 2005/26214
[Patent Literature 4] Japanese Patent Application Publication No. 2011-25013
[Patent Literature 5] Japanese Patent Application Publication No. 2013-165884
[Patent Literature 6] Japanese Translation of PCT Application No. 2016-502874
[Patent Literature 7] WO 2018/012605 and its English language equivalent EP 3485921
[Patent Literature 8] WO 2019/138583
[Patent Literature 9] U.S. Patent Application Publication No. 2012/0039959

### [Summary of Invention]

### [Problem to be Solved by Invention]

Under such circumstances, a new material for preventing adhesion has been desired. Preferably, there has been a need for a material for preventing adhesion that has at least one of the following performances: highly effective in preventing adhesions; capable of suppressing both adhesion of a wound and de novo adhesion; having no adverse effect on the living body applied; not interfering with healing of a wound; being able to be used for intestinal anastomosis or the like; allowing easy application via a trocar upon an endoscopic surgery; capable of being reattached to adjust the attached position; suitable for industrial production, and the like.

### [Means for Solving Problem]

The present inventors have gone through intensive studies on a material for preventing adhesion that has both advantages of a film (sheet)-like material for preventing adhesion and a spray (liquid/gel/powder)-type material for preventing adhesion in animal adhesion models assuming various clinical operations. As a result, the inventors have found that a material for preventing adhesion having a specific condition in a predetermined dissolution test does not only prevent adhesion at the surgical site but is also stable over a wide area of the applied region to have an excellent adhesion preventing effect and is suitable for industrial production, thereby accomplishing the present invention. The scope of the invention is defined by the claims. Any references in the description to methods of treatment refer to the products of the present invention for use in a method for treatment.

As a first aspect, the present invention provides a sheet-like material for preventing adhesiona as defined in claim 1.
In particular, provided is a sheet-like material for preventing adhesion which has been pressed and contains alginate, and at least a portion of which is crosslinked with a curing agent, the material for preventing adhesion satisfying (1) and (2) when a dissolution test is performed in which a sample cut into a substantial circle having a diameter of 8 mm is left to stand, via a mesh, on agar of a petri dish to which a physiological saline solution is added substantially to a top surface of the agar, the petri dish is shaken at an amplitude of 25 mm and 40 shakes/min, and a weight of the sample is measured least one arbitrary point in time:
(1) a time required for the weight of the sample to be less than 0.01 g is 5-36 hours from a start of the test; and
(2) a time required for the weight of the sample to reach a maximum weight is 10 hours or less from the start of the test.

In a preferred embodiment, the material for preventing adhesion comprises a first layer and a second layer.

In a further preferred embodiment of the material for preventing adhesion, the total amount of the alginate is 1.4 mg/cm² or more and 2.8 mg/cm² or less in terms of a weight of sodium alginate.

In a further preferred embodiment of the material for preventing adhesion, the curing agent is a calcium ion compound and a total amount of calcium is 0.14 mg/cm² or more and 0.30 mg/cm² or less in terms of a weight of calcium chloride.

In a further preferred embodiment of the material for preventing adhesion, the thickness of the sheet-like material for preventing adhesion is 100 µm or more and 500 µm or less.

In a further preferred embodiment of the material for preventing adhesion, the dissolution rate of the first layer is slower than that of the second layer.

In a further preferred embodiment, the material for preventing adhesion is a single layer.

As a second aspect, the present invention provides a method for producing the material for preventing adhesiona as defined in claim 8. In particular, provided is a method for producing the material for preventing adhesion of the fist aspect as defined above, the method comprising:
(1) a step of curing (gelating) an aqueous solution of a monovalent metal salt of alginic acid with a curing agent;
(2) optionally, a step of freezing the cured (gelated) monovalent metal salt of alginic acid;
(3) optionally, a step of curing (gelating) an aqueous solution of a monovalent metal salt of alginic acid with a curing agent on a first layer to form a second layer;
(4) optionally, a step of further repeating the step (2) and the step (3) to form a third layer; and
(5) a step of lyophilizing a resulting cured material (gel).

In a preferred embodiment of the method for producing the material for preventing adhesion, the monovalent metal salt of the alginic acid comprises at least a monovalent metal salt of alginic acid having a weight-average molecular weight of 100,000 or more.

As a third aspect, the present invention provides a sheet-like material for use in a method for preventing adhesiona as defined in claim 10. In particular, provided is a sheet like material for use in a method for preventing adhesion, the method comprising:
applying to a subject in need of adhesion prevention the sheet-like material for preventing adhesion which has been pressed and contains alginate, and at least a portion of which is crosslinked with a curing agent, the material for preventing adhesion satisfying (1) and (2) when a dissolution test is performed in which a sample cut into a substantial circle having a diameter of 8 mm is left to stand, via a mesh, on agar of a petri dish to which a physiological saline solution is added substantially to a top surface of the agar, the petri dish is shaken at an amplitude of 25 mm and 40 shakes/min, and a weight of the sample is measured at least one arbitrary point in time;
(1) a time required for the weight of the sample to be less than 0.01 g is 5-36 hours from a start of the test; and
(2) a time required for the weight of the sample to reach a maximum weight is 10 hours or less from the start of the test.

### [Effect of the Invention]

The present invention provides a new material for preventing adhesion. Notably itcan provide a material for preventing adhesion that has at least one of the following performances: highly effective in preventing adhesions; capable of suppressing both adhesion of a wound and de novo adhesion; has no adverse effect on the living body applied; does not interfere with healing of a wound; can be used for intestinal anastomosis; allows easy application via a trocar upon an endoscopic surgery; and capable of being reattached to adjust the attached position.

### [Brief Description of Drawings]

[Fig. 1]

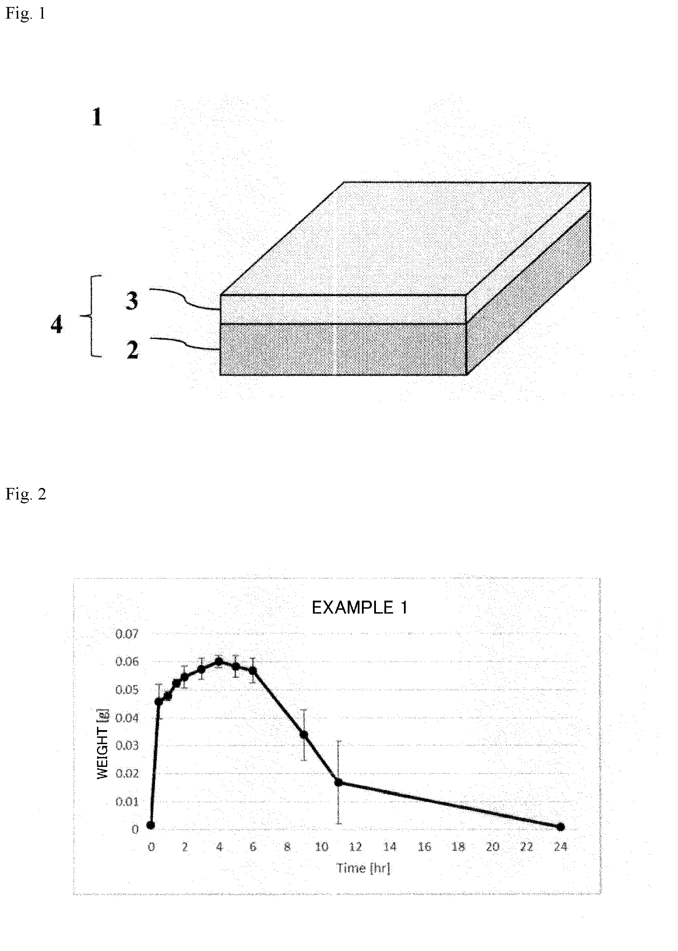

   Fig. 1 is a view showing an example of a material for preventing adhesion.
[Fig. 2]
   Fig. 2 is a graph showing results from a dissolution test of a material for preventing adhesion of Example 1.
[Fig. 3]

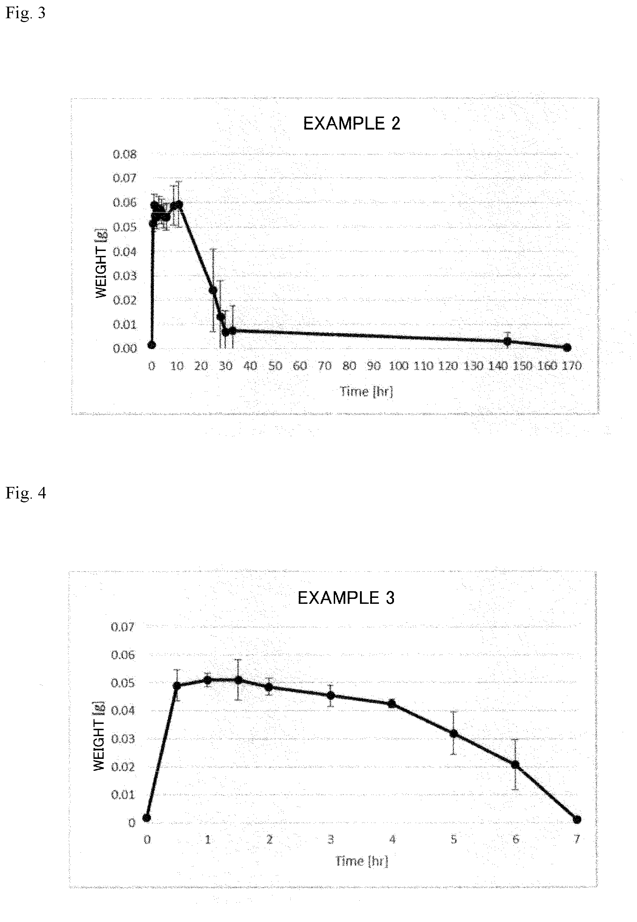

   Fig. 3 is a graph showing results from a dissolution test of a material for preventing adhesion of Example 2.
[Fig. 4]
   Fig. 4 is a graph showing results from a dissolution test of a material for preventing adhesion of Example 3.
[Fig. 5]

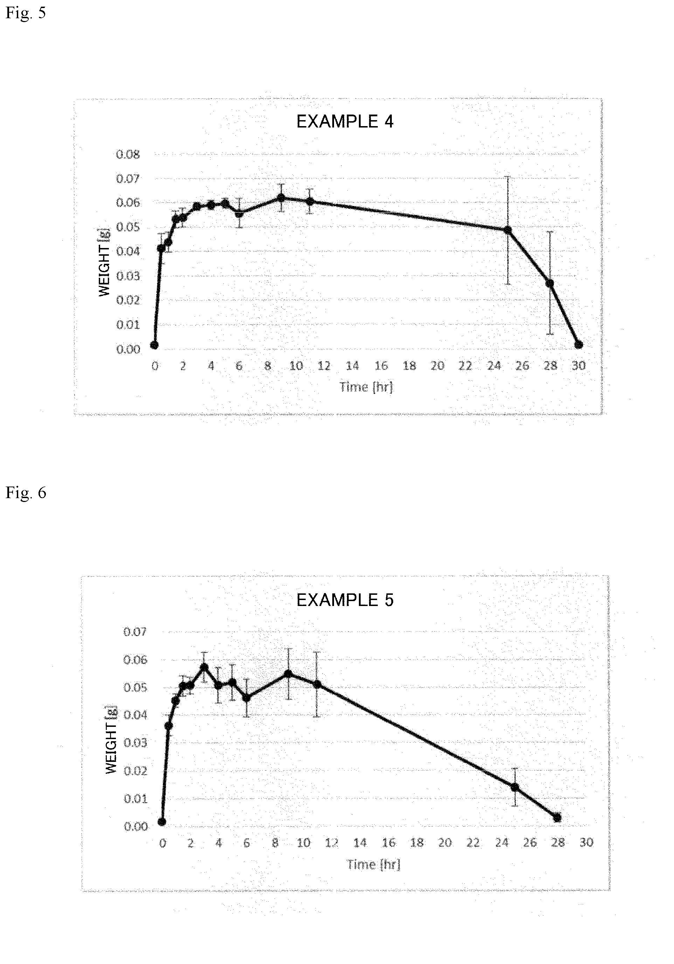

   Fig. 5 is a graph showing results from a dissolution test of a material for preventing adhesion of Example 4.
[Fig. 6]
   Fig. 6 is a graph showing results from a dissolution test of a material for preventing adhesion of Example 5.
[Fig. 7]

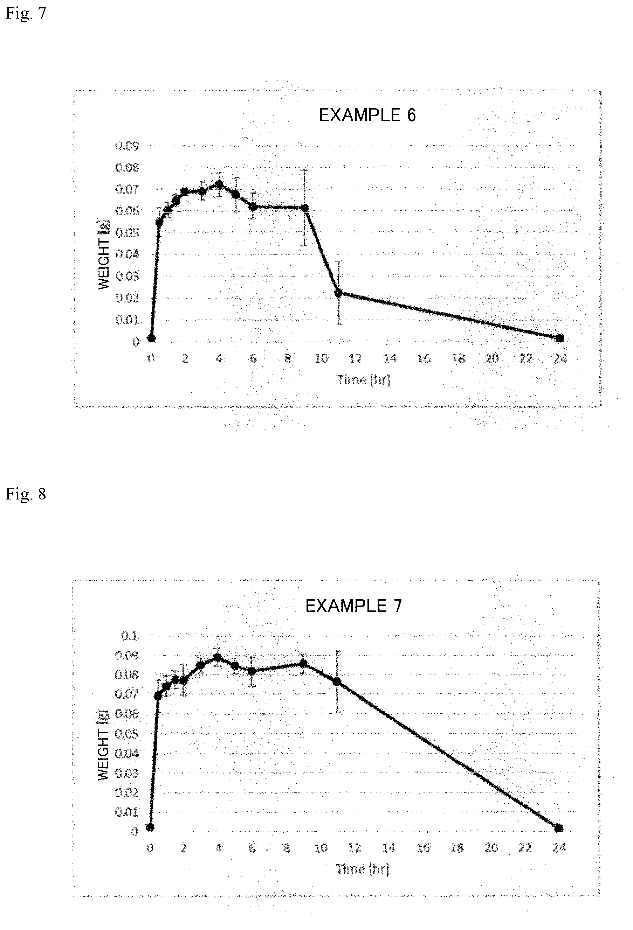

   Fig. 7 is a graph showing results from a dissolution test of a material for preventing adhesion of Example 6.
[Fig. 8]
   Fig. 8 is a graph showing results from a dissolution test of a material for preventing adhesion of Example 7.
[Fig. 9]

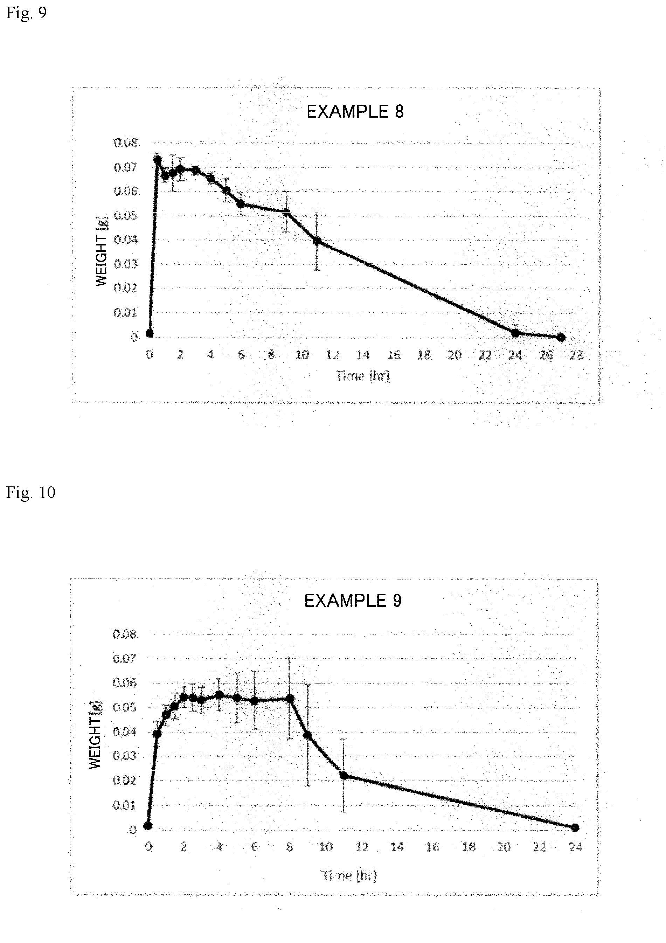

   Fig. 9 is a graph showing results from a dissolution test of a material for preventing adhesion of Example 8.
[Fig. 10]
   Fig. 10 is a graph showing results from a dissolution test of a material for preventing adhesion of Example 9.
[Fig. 11]

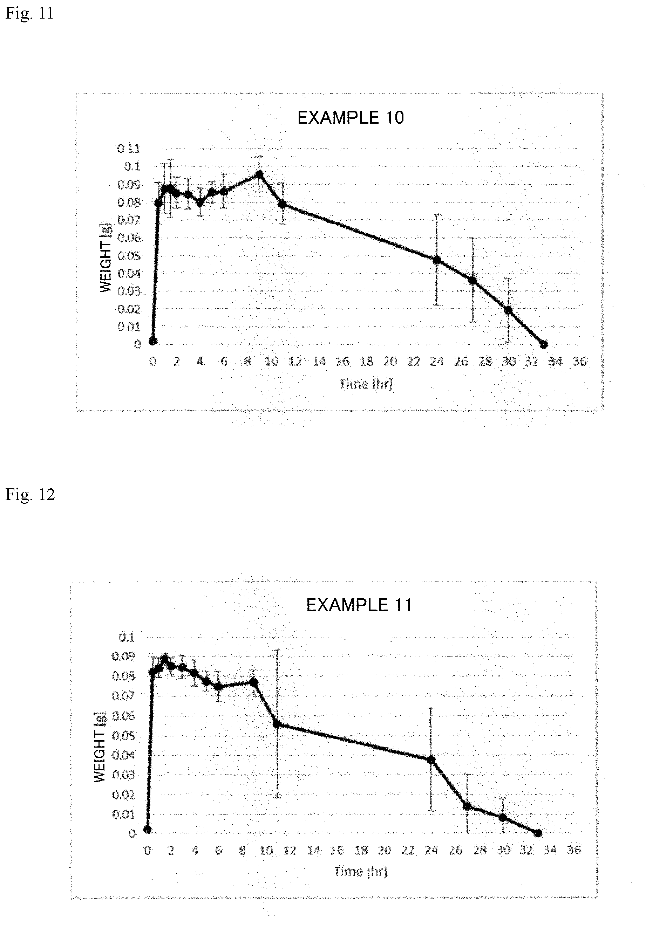

   Fig. 11 is a graph showing results from a dissolution test of a material for preventing adhesion of Example 10.
[Fig. 12]
   Fig. 12 is a graph showing results from a dissolution test of a material for preventing adhesion of Example 11.
[Fig. 13]

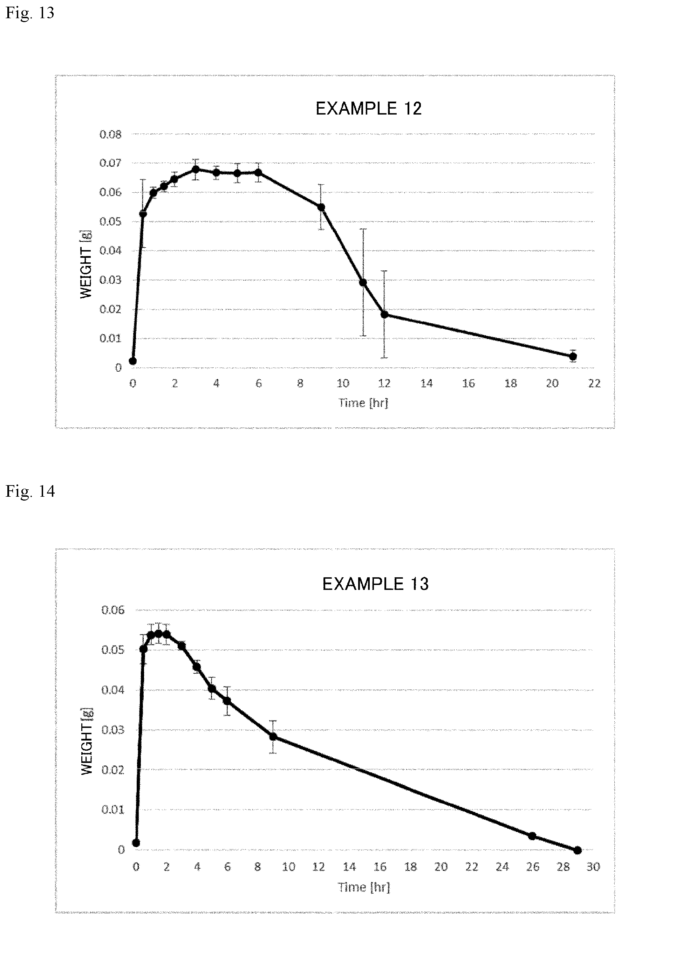

   Fig. 13 is a graph showing results from a dissolution test of a material for preventing adhesion of Example 12.
[Fig. 14]
   Fig. 14 is a graph showing results from a dissolution test of a material for preventing adhesion of Example 13.
[Fig. 15]

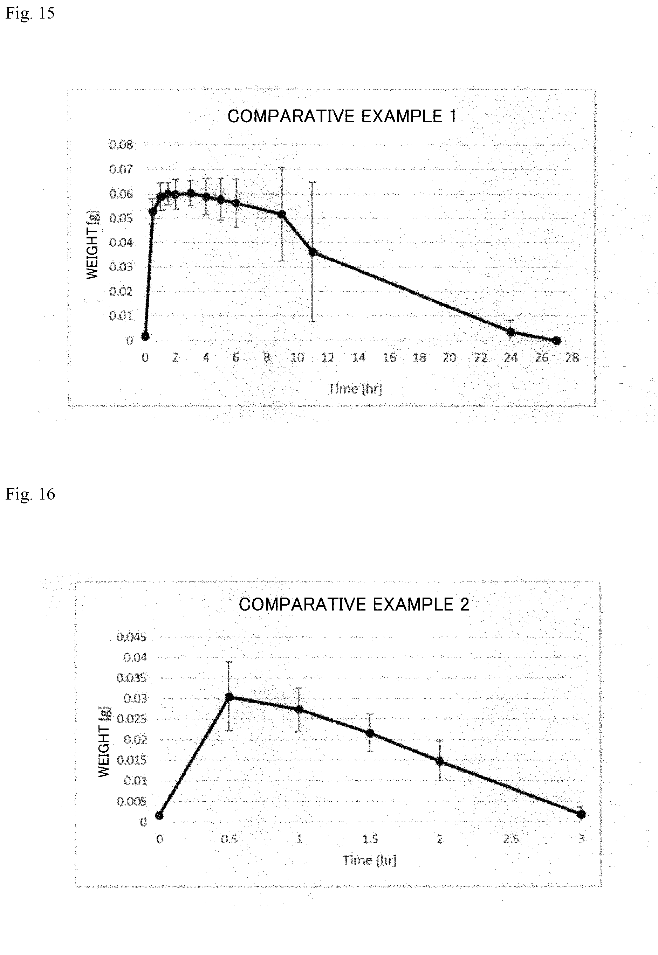

   Fig. 15 is a graph showing results from a dissolution test of a material for preventing adhesion of Comparative Example 1.
[Fig. 16]
   Fig. 16 is a graph showing results from a dissolution test of a material for preventing adhesion of Comparative Example 2.
[Fig. 17]

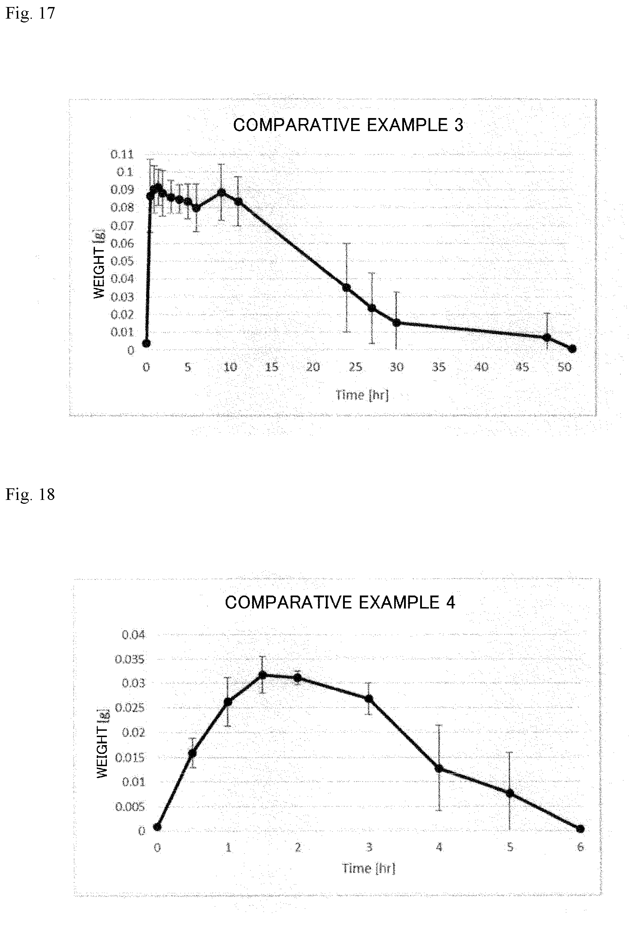

   Fig. 17 is a graph showing results from a dissolution test of a material for preventing adhesion of Comparative Example 3.
[Fig. 18]
   Fig. 18 is a graph showing results from a dissolution test of a material for preventing adhesion of Comparative Example 4.
[Fig. 19]

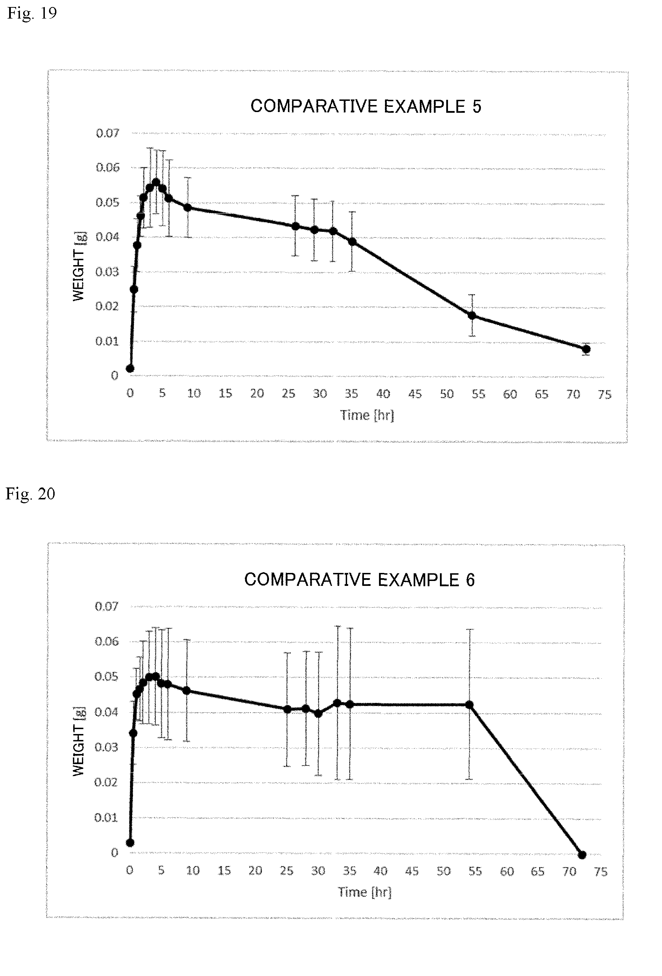

   Fig. 19 is a graph showing results from a dissolution test of a material for preventing adhesion of Comparative Example 5.
[Fig. 20]
   Fig. 20 is a graph showing results from a dissolution test of a material for preventing adhesion of Comparative Example 6.
[Fig. 21]

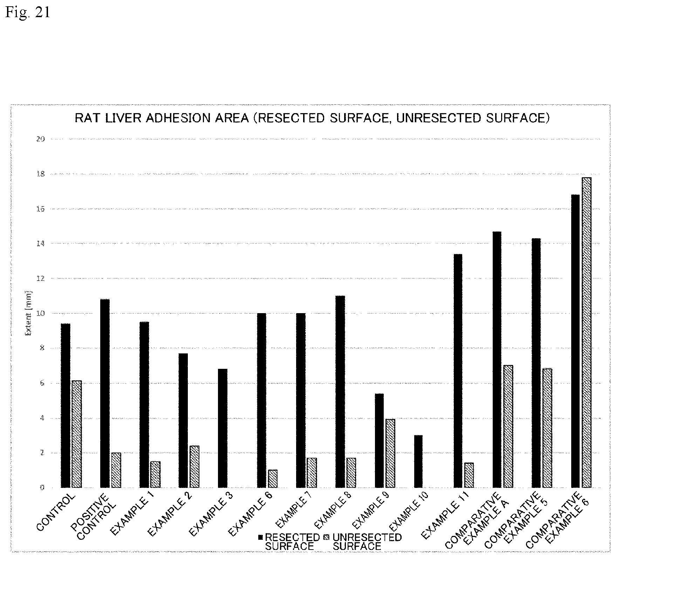

   Fig. 21 is diagrams showing evaluations of adhesion formation in resected rat hepatic models (unresected surfaces and resected surfaces).
[Fig. 22]

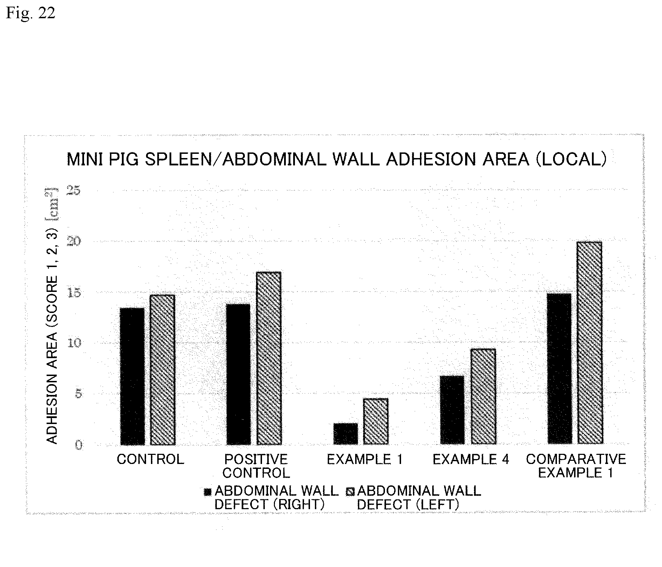

   Fig. 22 is diagrams showing evaluations of adhesion formation in mini pig spleen/abdominal wall adhesion models (local).
[Fig. 23]

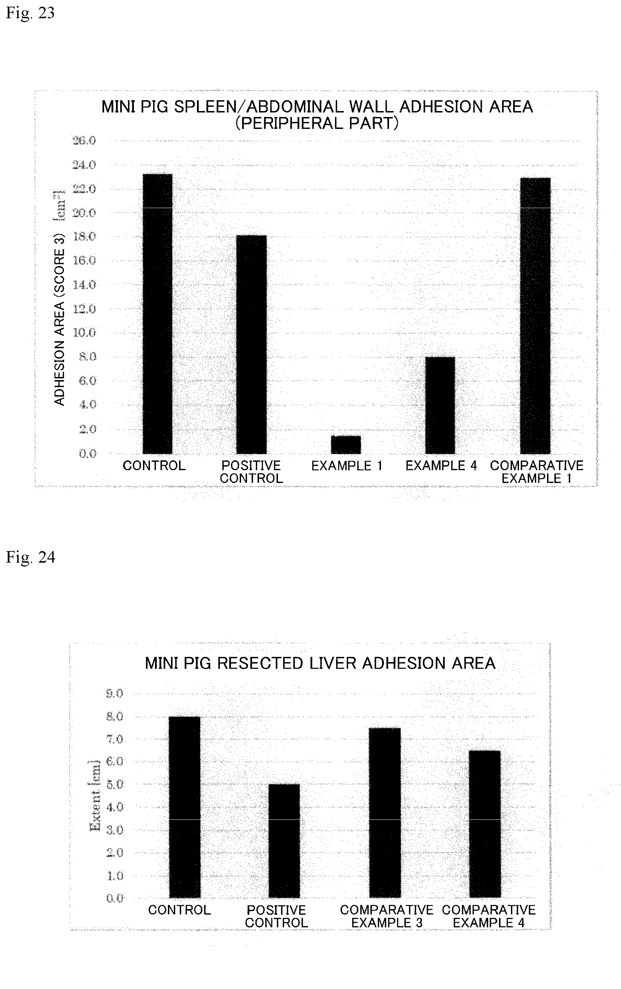

   Fig. 23 is diagrams showing evaluations of adhesion formation in mini pig spleen/abdominal wall adhesion models (peripheral parts).
[Fig. 24]
   Fig. 24 is diagrams showing evaluations of adhesion formation in two-stage resected mini pig hepatic models.
[Fig. 25]

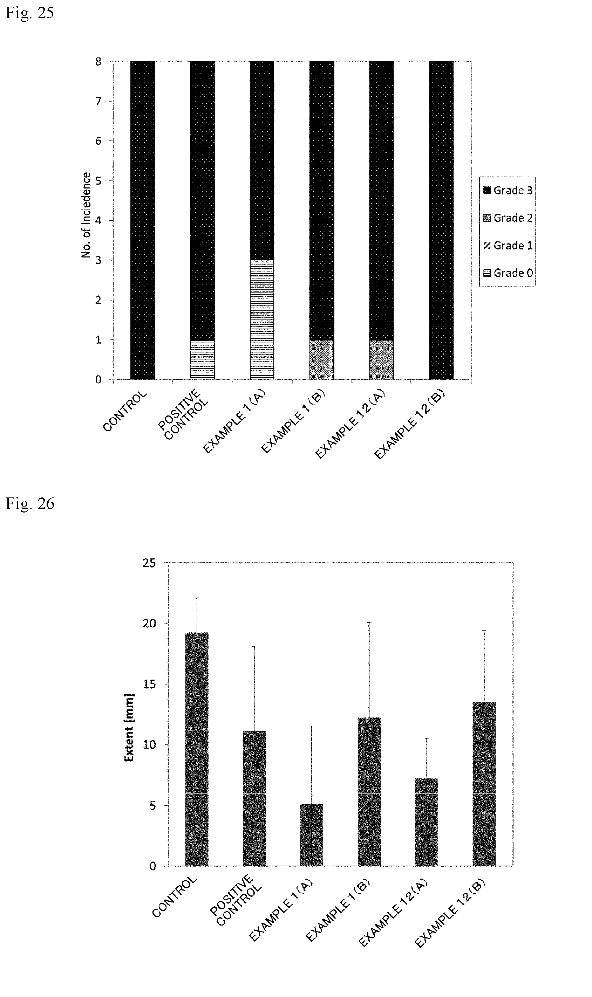

   Fig. 25 is diagrams showing evaluations of adhesion formation in partially resected rat hepatic models (Grade).
[Fig. 26]
   Fig. 26 is diagrams showing evaluations of adhesion formation in partially resected rat hepatic models (Extent).

### [Description of Embodiments]

Hereinafter, the present invention will be described in detail.

### 1. Adhesion prevention

"Adhesions" refer to a state where surfaces of tissues that should be separated from each other are connected or fused to each other via fibrous tissue. Adhesions are caused by an injury generated on a surface of a tissue upon a surgical operation, inflammation caused by an injury, and inflammation caused by drying of a tissue surface upon a surgical operation. Adhesions are formed in association with such injury or inflammation upon which an exudate containing fibrin is emitted on a surface of a tissue, where this exudate is organized such that the tissue surfaces are connected or fused.

"Adhesion prevention" means to reduce formation of adhesions. Adhesion prevention does not necessarily require complete prevention of formation of adhesions, and may apply as long as formation of adhesion is prevented compared to a state where the material for preventing adhesion is not applied. Specifically, "adhesion prevention" may also refer to as amelioration of adhesions, which may mean, for example, amelioration of at least one selected from frequency, area and degree of the adhesions. "Adhesion prevention" may be, for example, lowering of the average adhesion extent as compared to an average adhesion extent without application of the material for preventing adhesion when adhesion extent is evaluated as described in the example. "Adhesion prevention" preferably refers to prevention of adhesion resulting from a surgical operation, and more preferably refers to prevention of peritoneal adhesion resulting from a surgical operation. Specifically, "adhesion prevention" preferably refers to adhesion prevention following a surgery.

In addition, targeted adhesions may be an adhesion of a site of a target organ resected upon a surgery and de novo adhesions (adhesions formed with various sites in the periphery, the abdominal cavity and the body other than the surgical site).

### 2. Material for preventing adhesion

Here, the following material for preventing adhesion is provided.

There is provided a sheet-like material for preventing adhesion of the first aspect of the invention as defined hereinbefore, which has been pressed and contains alginate, and at least a portion of which is crosslinked with a curing agent, and in which the material for preventing adhesion satisfies (1) and (2) when a dissolution test is performed in which a sample having a weight of about 2 mg and cut into a substantial circle having a diameter of 8 mm is left to stand, via a mesh, on agar of a petri dish to which a physiological saline solution is added substantially to a top surface of the agar, the petri dish is shaken at an amplitude of 25 mm and 40 shakes/min., and a weight of the sample is measured at least one arbitrary point in time;
(1) a time required for the weight of the sample to be less than 0.01 g is 5-36 hours from a start of the test, and
(2) a time required for the weight of the sample to reach a maximum weight is 10 hours or less from the start of the test.

The shape of the material for preventing adhesion is not particularly limited as long as the shape is a sheet shape and may suitably be selected considering the area, shape, unevenness and the like of the surface to be applied. The shape of the material for preventing adhesion may be, for example, a rectangular (including square) plate or may have a shape such as a disc. The shape is preferably a rectangular (including square) plate or disc. If it is a plate or a disc, the material for preventing adhesion can further be cut in accordance with the area, shape, unevenness of the surface applied before being applied to the surface. The material for preventing adhesion has been pressed. Specifically, the pressed material for preventing adhesion is obtained by pressing a sponge-like material. The term "sponge-like" refers to a porous state. A porous sponge-like composition is obtained by lyophilizing a gel cured by a curing agent. Pressing can be performed by holding and applying pressure on the laminate manually or with a press machine. By pressing (applying pressure on) the sponge-like composition, pores are crushed, the sponge-like composition deforms and becomes a sheet-like composition. Pressing has effect on the moisture absorption and dissolution time of the sheet-like composition. Generally employed steps such as compression and thinning are also included as pressing mentioned herein. Examples of the pressure adopted for pressing include 1 kPa to 100 MPa, more preferably 10 kPa to 80 MPa, and still more preferably 100 kPa to 60 Mpa. Manual pressing is performed with a means that can apply uniform pressure onto the laminate by pressing it with a hand, for example, an acrylic ruler, an acrylic plate, a glass plate, or a metal plate. Moreover, an example of the press machine used includes a hot press machine (AH-1T from AS ONE Corporation).

The height (thickness) of the pressed material for preventing adhesion is preferably 100-500 µm, more preferably 150-450 µm, and still more preferably 200-400 µm.

A material for preventing adhesion of a more preferable aspect is a rectangular (including square) plate and has such a height (thickness), and, additionally, the length and the width are 1 mm-300 mm × 1 mm-300 mm, more preferably 3 mm-200 mm × 3 mm-200 mm, and still more preferably 5 mm-150 mm × 5 mm-150 mm, respectively.

A material for preventing adhesion of a still more preferable different aspect is a disc and has such a height (thickness), and, additionally, the diameter is 10 mm-300 mm, more preferably 20 mm-200 mm, and still more preferably 30 mm-150 mm. A material pressed to be a sheet can also be rolled or folded and is appropriately soft and resilient enough to be easily restored to a sheet shape after rolled.

In several aspects, the thickness of the pressed material for preventing adhesion is uniform. In addition, in several different aspects, the thickness may not be uniform and the pressed material for preventing adhesion may have a slope structure with one thicker end and the other thinner end. Herein, a pressed sponge-like composition containing alginate is also referred to as an alginic acid sheet.

The material for preventing adhesion contains alginate, at least a portion of which is crosslinked with a curing agent. The material for preventing adhesion preferably contains a monovalent metal salt of alginic acid. The "curing agent" and the "monovalent metal salt of alginic acid" will be described hereinbelow.

The dissolution test of the material for preventing adhesion will be described in detail. The material for preventing adhesion has predetermined conditions when the following dissolution test has been performed.

In the dissolution test, a petri dish, agar, a physiological saline solution, a sample, a mesh, and a shaker (shaking machine) are used. The petri dish is not particularly limited as long as the agar and the mesh on which the sample are placed can be put into the petri dish, and, for example, a dish having a diameter of 10 cm (product code: 3020-100, manufactured by IWAKI & Co., Ltd.) or the like may be used. As the agar, for example, a 2% agar solution solidified in a tray may be used, and the concentration can be suitably changed. The sample is obtained by cutting the material for preventing adhesion into a substantial circle having a diameter of 8 mm. Although a method for cutting the sample is not particularly limited, for example, a sample having the size and shape can be obtained using an 8 mm-diameter biopsy punch.

The weight of the sample measured at this time is regarded as the weight of the sample at the start of the measurement.

Next, agar having a thickness of 0.7 cm cut into predetermined sizes (specifically, 1 cm × 1 cm sizes) is left to stand on the petri dish, and the physiological saline solution is poured into the petri dish. The amount of the physiological saline solution is an amount at which the physiological saline solution reaches substantially the top surface of the agar when poured into the petri dish.

Next, the sample placed on the mesh is left to stand on the agar put into the petri dish, set in the shaker (shaking machine), and starts to be shaken. Here, this start of shaking may be referred to as "the start of the test". The mesh used herein is not particularly limited, and, for example, a mesh made by cutting off the side surface of a cell strainer (100 µm, FALCON) may be used. The tare weight of the mesh is measured in advance using an electronic balance.

The shaker is also not particularly limited, and, for example, TRIPLE SHAKER NR-80 (manufactured by TAITEC Corporation) is illustrated. The shaking is performed by shaking the petri dish at an amplitude of 25 mm and 40 shakes/min. Here, since the water surface of the physiological saline solution is substantially as high as the top surface of the agar, the physiological saline solution is splashed to the sample occasionally during the shaking. Regarding the temperature during the shaking, the shaking is performed at room temperature.

Next, the weight of the sample is measured at least one arbitrary point in time from the start of the test. Then, the test is completed once the complete dissolution of the measurement sample is visually confirmed. Here, "at least one arbitrary point in time" is, for example, at least one arbitrary point in time from the start of the test to the complete dissolution of the measurement sample, and examples thereof include at least one arbitrary point in time of 0.5 hours, 1 hour, 1.5 hours, 2 hours, 2.5 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 30 hours, 33 hours, 48 hours, 49 hours, 51 hours, 52 hours, 54 hours, 55 hours, 58 hours, 144 hours, and 168 hours from the start of the test.

The weight of the sample is measured every hour using the electronic balance after the mesh is taken out and moisture is wiped off. After the measurement of the weight, the mesh is put back onto the agar in the petri dish, and a specified amount, for example, 200 µL of the physiological saline solution is added.

In more detail, the dissolution test can be performed by a method disclosed in an example to be described below.

The material for preventing adhesion satisfies the following (1) and (2) when the dissolution test has been performed.
(1) A time required for the weight of the sample to be less than 0.01 g is 5-36 hours from a start of the test, and
(2) a time required for the weight of the sample to reach a maximum weight is 10 hours or less from the start of the test.

For example, in subject living bodies such as human beings, materials for preventing adhesion of several aspects absorb moisture to swell, are dissolving such that the amount of moisture absorbed reaches a saturation state, and all dissolves in the end.

In this case, (1) indicates that the time taken for the material for preventing adhesion to completely dissolve in a living body is not too short and the material for preventing adhesion does not continuously remain long.

In addition, (2) indicates that the material for preventing adhesion rapidly absorbs water to swell in a living body.

Regarding (1), "5-36 hours" can be, for example, 5-36 hours, 10-30 hours, 12-24 hours, or the like from the start of the test.

In addition, regarding (2), "10 hours or less" can be, for example, 10 hours or less, 9.5 hours or less, 9 hours or less, 8.5 hours or less, 8 hours or less, 7 hours or less, 6 hours or less, or the like.

Here, "the time required for the weight of the sample to reach the maximum weight" in (2) becomes a shorter time than "the time required for the weight of the sample to be less than 0.01 g" in (1). In other words, "the time required for the weight of the sample to be less than 0.01 g" in (1) becomes a longer time than "the time required for the weight of the sample to reach the maximum weight" in (2).

Herein, a sign "-" used for a numerical range refers to "the lower limit value to the upper limit value" where the numerical values on both sides of the sign are inclusive in the range.

The material for preventing adhesion is made to satisfy (1) and (2), for example, by adjusting the total weight of the curing agent, by adjusting the total weight of the monovalent metal salt of alginic acid, by adjusting the distribution ratio of the curing agent in the upper layer to the lower layer, by adjusting the distribution ratio of the monovalent metal salt of alginic acid in the upper layer to the lower layer, by adjusting the thickness after pressing, or the like.

Regarding the adjustment of the total weight of the curing agent, for example, when a calcium ion compound is used as the curing agent, the amount of the curing agent is made to be 0.14 mg/cm² or more in terms of the weight of calcium chloride, whereby the local and de-novo adhesion preventing effect can be obtained, and the condition of (1) can be satisfied. In addition, the amount of the curing agent is made to be 0.30 mg/cm² or less in terms of the weight of calcium chloride, whereby a homogeneous material for preventing adhesion can be obtained at the time of production, industrial productivity improves, and the conditions of (1) and (2) can be satisfied.

Regarding the adjustment of the total weight of the monovalent metal salt of alginic acid, for example, the total weight of the monovalent metal salt of alginic acid is made to be 1.4 mg/cm² or more in terms of the weight of sodium alginate, whereby the local and de-novo adhesion preventing effect can be obtained, and the condition of (1) can be satisfied (the dissolution rate of the material for preventing adhesion can be made to be as slow as an appropriate rate). In addition, the total weight of the monovalent metal salt of alginic acid is made to be 2.8 mg/cm² or less in terms of the weight of sodium alginate, whereby a material for preventing adhesion, excessive remaining of which in a living body is ameliorated can be obtained, and, additionally, the condition of (1) can be satisfied (the dissolution rate of the material for preventing adhesion can be made to be as fast as an appropriate rate).

Regarding the adjustment of the distribution ratio of the curing agent in the upper layer to the lower layer, for example, the distribution ratio of the curing agent in the upper layer to the lower layer is made to be 0.1-1.2, whereby the local and de-novo adhesion preventing effect can be obtained. In several aspects of the present invention, the distribution ratio of the curing agent in the upper layer to the lower layer is preferably 0.1-0.6 and more preferably 0.2-0.4.

Regarding the adjustment of the distribution ratio of the monovalent metal salt of alginic acid in the upper layer to the lower layer, for example, the distribution ratio of the monovalent metal salt of alginic acid in the upper layer to the lower layer is made to be 1-3, whereby the local and de-novo adhesion preventing effect can be obtained. In several aspects of the present invention, the distribution ratio of the monovalent metal salt of alginic acid in the upper layer to the lower layer is preferably 2-3 and more preferably 3.

Regarding the adjustment of the thickness after pressing, for example, the thickness after pressing is made to be 100-500 µm, whereby good operability such as insertion via a trocar upon an endoscopic surgery becoming possible can be obtained. In addition, the thickness after pressing is made to be in the above range, the conditions of (1) and (2) can be satisfied.

When the curing agent is a calcium ion compound, the total amount of the curing agent contained in 72 cm² of the material for preventing adhesion is set to, for example, 10 mg or more and less than 22 mg, preferably 13 mg-20 mg, and more preferably 17 mg-19 mg in terms of the weight of calcium chloride.

In addition, in several aspects, when the curing agent is a calcium ion compound, the total amount of the curing agent contained in one square centimeter of the material for preventing adhesion is set to, for example, 0.14 mg-0.30 mg, preferably 0.18 mg-0.28 mg, and more preferably 0.24 mg-0.27 mg.

"Total amount" can be rephrased as "amount used", "amount blended", "amount added", and the like. **In** addition, "total amount" means the amount when the thickness of the material for preventing adhesion is set to 100-500 µm.

The material for preventing adhesion may not have a layered structure (single layer), may have a layered structure, or may have a structure of a laminate of two or more layers. The laminate of two or more layers is, for example, a laminate of two layers, three layers, four layers, or five layers, preferably a laminate of two layers or three layers, and more preferably a laminate of two layers. When the material for preventing adhesion is a laminate, the material for preventing adhesion may have a structure in which there is a clear boundary between the layers or may have a structure in which there is no clear boundary between the layers.

An example of the material for preventing adhesion having a two-layer structure is shown in Fig. 1. A material for preventing adhesion 1 includes a laminate 4 including a first layer 2 and a second layer 3. The laminate 4 is obtained by pressing a sponge-like laminate as necessary. The first layer 2 and the second layer 3 are each a sponge-like layer before pressing.

The "first layer" refers to a layer that becomes a lower layer when the laminate is applied to a subject, namely, a layer that makes contact with a surface of a tissue for application in the subject. The "second layer" refers to a layer that becomes an upper layer when the laminate is applied to a subject, namely, a layer that does not make contact with a surface of a tissue for application in the subject. The material for preventing adhesion is biocompatible. The phrase "biocompatible" means that it can be placed on a surface of a tissue to be applied as a medical material.

A biocompatible laminate used as a material for preventing adhesion may have, in addition to the above-described first and second layers, a third layer containing any component.

In several aspects, materials for preventing adhesion include a biocompatible laminate which includes a first layer and a second layer each containing a monovalent metal salt of alginic acid which is at least partially crosslinked with a curing agent, and the dissolution rate of the first layer is slower than that of the second layer. The dissolution rate of the first layer may be made slower than the dissolution rate of the second layer, for example, by making the weight-average molecular weight of the monovalent metal salt of alginic acid in the first layer to be higher than that in the second layer, or by making the crosslinking degree of the monovalent metal salt of alginic acid in the first layer to be higher than that in the second layer by changing the type of the curing agent, by changing the concentration of the curing agent, or the like.

When the weight-average molecular weight of the monovalent metal salt of alginic acid in the first layer is made to be higher than that in the second layer, the weight-average molecular weights of the monovalent metal salts of alginic acid used in the first layer and in the second layer are each made to be, for example, 10,000-2,000,000 and 1,000-1,000,000. Such weight-average molecular weights are measured by GPC-MALS method following a decrosslinking treatment, for example, following dissolution in a solution of a chelating agent.

The monovalent metal salt of alginic acid may be used as a combination of a plurality of monovalent metal salts of alginic acid having different weight-average molecular weights. For example, in the first layer, a combination of a plurality of monovalent metal salts of alginic acid having different weight-average molecular weights in a range of 10,000-2,000,000 may be used, and/or, in the second layer, a combination of a plurality of monovalent metal salts of alginic acid having different weight-average molecular weights in a range of 1,000-1,000,000 may be used. The number of the monovalent metal salts of alginic acid to be combined is not particularly limited, and may be, for example, 2, 3, 4, 5, 6, 7, 8, 9, 10 or more in one layer. The monovalent metal salts of alginic acid may be a combination of different types of salts having different weight-average molecular weights or a combination of the same type of salts having different weight-average molecular weights. The ratio of the combination is also not particularly limited. For example, when two types of salts are combined, the ratio thereof may be 1:100-100:1, 1:50-50:1, 1:25-25:1, 1:10-10:1, 1:5-5:1, 1:4-4:1, 1:3-3:1, 1:2-2:1, 1:1 or the like.

In several aspects, the total amount of the monovalent metal salt of alginic acid contained in 72 cm² of the material for preventing adhesion is set to, for example, 100 mg-200 mg, preferably 120 mg-180 mg, and more preferably 150 mg-170 mg in terms of the weight of sodium alginate.

In addition, in several different aspects, the total amount of the monovalent metal salt of alginic acid contained in one square centimeter of the material for preventing adhesion is set to, for example, 1.4 mg-2.8 mg, preferably 1.7 mg-2.5 mg, and more preferably 2.1 mg-2.4 mg in terms of the weight of sodium alginate.

In several aspects of the present invention, it is preferable that the total amount of the alginate contained in one square centimeter of the material for preventing adhesion is 2.1 mg-2.4 mg in terms of the weight of sodium alginate, the distribution ratio of the alginate in the second layer (or the upper layer) to the first layer (or the lower layer) is 2-3, the curing agent is a calcium ion compound, the total amount of the curing agent contained in one square centimeter of the material for preventing adhesion is 0.24 mg-0.27 mg in terms of the weight of calcium chloride, and the distribution ratio of the curing agent in the second layer (or the upper layer) to the first layer (or the lower layer) is 0.2-0.4.

In several aspects, the monovalent metal salt of the alginic acid used for the production of the material for preventing adhesion contains at least alginic acid having a weight-average molecular weight of 100,000 or more. This makes it possible to increase the physical strength of the material for preventing adhesion.

A material for preventing adhesion of a preferable aspect is highly flexible and hard to break as compared to Seprafilm (trade name).

### 3. Alginate

The alginate used as a raw material of the material for preventing adhesion of the present invention is a monovalent metal salt of alginic acid.

A "monovalent metal salt of alginic acid" is a water-soluble salt that is formed through ion exchange between a hydrogen atom of carboxylic acid at position 6 of mannuronic acid and/or guluronic acid, which is a constituent sugar of alginic acid and a monovalent metal ion such as Na⁺ or K⁺. Specific examples of monovalent metal salts of alginic acid include sodium alginate and potassium alginate, while sodium alginate that can be obtained as a commercially available product is particularly preferable. A solution of a monovalent metal salt of alginic acid forms a gel when mixed with a curing agent.

"Alginic acid" used in the present invention is a biodegradable polymeric polysaccharide, which is a polymer resulting from linear polymerization of two types of uronic acids called D-mannuronic acid (M) and L-guluronic acid (G). More specifically, alginic acid is a block copolymer which has a homopolymer fraction of D-mannuronic acid (MM fraction), a homopolymer fraction of L-guluronic acid (GG fraction) and a fraction having randomly arranged D-mannuronic acids and L-guluronic acids (MG fraction), arbitrarily linked together. A composite ratio of D-mannuronic acid to L-guluronic acid (M/G ratio) of alginic acid varies primarily according to the type of a biological origin such as seaweed, and is affected by the habitat and seasons of the biological origin. The M/G ratio widely ranges from about 0.4 that is rich in G to about 5 that is rich in M.

Although an alginic acid that is extracted from a brown alga initially has a large molecular weight, the molecular weight gradually becomes smaller during the processes of heat drying, purification and the like. Alginic acids having different molecular weights can be produced by techniques like management of conditions such as the temperature or the like during the production steps, selection of the brown alga as the raw material, fractionation based on molecular weights during the production process and the like. Furthermore, an alginic acid having a molecular weight of interest can be obtained by mixing with an alginic acid from other lot having a different molecular weight.

A monovalent metal salt of alginic acid used in the present invention is preferably subjected to a low endotoxin treatment. The low endotoxin treatment can be performed according to a publicly known method or a method pursuant thereto. For example, the treatment can be performed according to the method of Suga et al. involving purification of sodium hyaluronate (see, for example, Japanese Patent Application Publication No. H09-324001), the method of Yoshida et al., involving purification of β1,3-glucan (see, for example, Japanese Patent Application Publication No. H08-269102), the method of William et al. involving purification of a biopolymer salt such as alginate or gellan gum (see, for example, Japanese Translation of PCT Application No. 2002-530440), the method of James et al. involving purification of a polysaccharide (see, for example, WO 93/13136), the method of Lewis et al. (see, for example, specification of U. S. Patent No. 5589591), the method of Hermanfranck et al. involving purification of alginate (see, for example, Appl Microbiol Biotechnol (1994) 40:638-643), or a method pursuant thereto. The low endotoxin treatment of the present invention is not limited thereto, and can be performed by a publicly known method such as washing, filtration with a filter (such as an endotoxin-removing filter or an electrically-charged filter), ultrafiltration, purification with a column (such as an endotoxin adsorption affinity column, a gel filtration column or an ion-exchange resin column), adsorption to a hydrophobic substance, a resin or activated charcoal, a treatment with an organic solvent (extraction with an organic solvent, deposition/precipitation through addition of an organic solvent, or the like), a surfactant treatment (see, for example, Japanese Patent Application Publication No. 2005-036036), or a suitable combination thereof. The steps in these treatments may suitably be combined with a publicly known method such as centrifugation. Preferably, the treatment is suitably selected according to the type of the alginic acid.

An endotoxin level can be confirmed according to a publicly known method. For example, it can be measured by a method using a limulus reagent (LAL), or a method using Endospecy (registered trademark) ES-24S set (Seikagaku Corporation).

Although a method for treating endotoxin of a monovalent metal salt of alginic acid used in the present invention is not particularly limited, the resulting endotoxin content of a bioabsorbable polysaccharide is preferably 500 endotoxin unit (EU)/g or less, more preferably 100 EU/g or less, still more preferably 50 EU/g or less, and particularly preferably 30 EU/g or less upon an endotoxin measurement using a limulus reagent (LAL). Sodium alginate that has been subjected to a low endotoxin treatment is available, for example, as a commercially available product such as Sea Matrix (registered trademark) (Mochida Pharmaceutical Co., Ltd.) and PRONOVA^{™} UP LVG (FMC BioPolymer). In addition, AL10, AL20, AL100, and AL500, which are the low-endotoxin sodium alginate described in WO 2018/012605 and WO 2019/138583 can also be used.

In addition, in several aspects, when the material for preventing adhesion is made to have a multilayer structure having a first layer and a second layer, the ratio of the amounts of the monovalent metal salts of alginic acid used in the first layer and the second layer (weight ratio) is preferably 1:20-20:1, more preferably 1:5-5:1, and still more preferably 1:3-3:1.

Herein, "alginic acid or a salt thereof" may sometimes collectively be referred to as "alginic acid".

### 4. Curing agent (crosslinking agent)

The material for preventing adhesion contains alginate, at least a portion of which is crosslinked with a curing agent. When the material for preventing adhesion has a multilayer structure, several layers may contain alginate crosslinked with a curing agent and the other layers may not contain alginate crosslinked with a curing agent or all layers may contain alginate crosslinked with a curing agent. When the material for preventing adhesion is a two-layer structure having a first layer and a second layer, any one of the first layer and the second layer may contain alginate crosslinked with a curing agent (that is, any one of the first layer and the second layer may not contain alginate crosslinked with a curing agent) or both the first layer and the second layer may contain alginate crosslinked with a curing agent.

The curing agent allows hardening of the monovalent metal salt of alginic acid by crosslinking a solution of the monovalent metal salt of alginic acid. Examples of the curing agent include bivalent or higher metal ion compounds of Ca²⁺, Mg²⁺, Ba²⁺, Sr²⁺, Zn²⁺ and Fe³⁺ and crosslinking reagents that have two to four amino groups within their molecules. These form a salt with alginic acid and, simultaneously, form an ionic crosslink. This crosslink is reversible and can be decrosslinked by, for example, a treatment with EDTA or the like. As the curing agent, more specifically, examples of bivalent or higher metal ion compounds include CaCl₂, MgCl₂, CaSO₄, ZnCl₂, FeCl₃, BaCl₂ and SrCl₂ (preferably, CaCl₂, CaSO₄, ZnCl₂, SrCl₂, FeCl₃, BaCl₂, etc.), while examples of crosslinking reagents having two to four amino groups within their molecules include diaminoalkanes optionally having a lysyl group (-COCH(NH₂)-(CH₂)₄-NH₂) on a nitrogen atom, that is, diaminoalkane and derivatives thereof that form lysyl amino groups by substituting an amino group with a lysyl group, specific examples being diaminoethane, diaminopropane and N-(lysyl)-diaminoethane. Among them, the material for preventing adhesion preferably contains a curing agent containing CaCl₂ as the bivalent or higher metal ion compound.

The amount of the curing agent used is preferably adjusted suitably in accordance with the amount or the molecular weight of the monovalent metal salt of alginic acid used. The amount of the curing agent used is, for example, in case of calcium chloride, 1.26 µmol/cm²-2.70 µmol/cm², preferably 1.62 µmol/cm²-2.52 µmol/cm², and more preferably 2.16 µmol/cm²-2.43 µmol/cm².

In addition, in several aspects, when the material for preventing adhesion is made to have a multilayer structure having a first layer and a second layer, the ratio of the amounts of the curing agent used in the first layer and the second layer (weight ratio) is preferably 1:10-10:1, more preferably 1:5-5:1, and still more preferably 1:3-3:1.

In addition, in several aspects, when the curing agent is a calcium ion compound, the ratio of the amount of the monovalent metal salt of alginic acid used to the total amount of the curing agent (weight ratio) is preferably 1:20-20:1 and more preferably 1:16-16:1.

### 5. Method for producing material for preventing adhesion

The material for preventing adhesion may, for example, be produced through the following steps: A material for preventing adhesion not having a layered structure (single layer) may be produced by the steps (1) and (5). In addition, a material for preventing adhesion having a two-layer structure (laminate structure) may be produced by the steps (1), (2), (3), and (5) or the steps (1), (3), and (5), and adhesion prevention having a three-layer structure (laminate structure) may be produced by the steps (1), (2), (3), (4), and (5) or the steps (1), (3), (4), and (5).
(1) Curing (gelating) an aqueous solution of a monovalent metal salt of alginic acid with a curing agent,
(2) optionally, freezing the cured (gelated) monovalent metal salt of alginic acid,
(3) optionally, curing (gelating) an aqueous solution of a monovalent metal salt of alginic acid with a curing agent on a first layer to form a second layer,
(4) optionally, further repeating the step (2) and the step (3) to form a third layer, and
(5) lyophilizing a resulting cured material (gel).

Specifically, a method for producing a laminated material for preventing adhesion having a two-layer structure will be described.

In step (1) above, first, a solution of a monovalent metal salt of alginic acid (hereinafter, referred to as "the first alginate") and a solution of a curing agent are prepared. The solution of the first alginate and the solution of the curing agent can be prepared according to a publicly known method or a method pursuant thereto. While the solvent can be any solvent as long as it is biocompatible, it is preferably an aqueous solvent, for example, purified water, pure water (e.g., distilled water, ion-exchanged water), Milli-Q water, physiological saline solution, phosphate buffered saline solution and DMSO, and more preferably pure water. The solvent is preferably one that has been sterilized and that has been subjected to a low endotoxin treatment.

Then, the solution of the first alginate and the solution of the curing agent can be mixed to cure (gelate) the first alginate. The mixing can be performed using, for example, a plate-like container.

In step (2) above, the first alginate cured in step (1) is frozen by a common method as desired. Freezing prior to step (3) can decrease the mixed proportion of the first layer and the second layer. The temperature and time of freezing may be, for example, -20°C for four hours.

In step (3) above, first, a solution of a monovalent metal salt of alginic acid (hereinafter, referred to as "the second alginate") and a solution of a curing agent are prepared. The solution of the second alginate and the solution of the curing agent can be prepared according to a publicly known method or a method pursuant thereto. The solvent is similar to that described for step (1) above.

Then, the solution of the second alginate and the solution of the curing agent can be mixed on the first layer to cure (gelate) the second alginate.

In step (5) above, the laminate obtained in step (3) above is lyophilized.

Lyophilizing can be performed by a publicly known method. First, the laminate is frozen, and the temperature and time of freezing may be, for example, -20°C for four hours. Conditions for drying can suitably be adjusted, and drying may include a primary drying step, a secondary drying step and the like.

Through these steps, a biocompatible material for preventing adhesion comprising a first layer containing a first alginate and a curing agent and a second layer containing a second alginate and a curing agent can be obtained.

Regarding the order of making the lower layer (a layer that comes into close contact with a living body damage site) and the upper layer (body cavity (abdominal cavity or the like) side) as the material for preventing adhesion, any layer may be made as the first layer and gelated first.

In addition, alternatively, the first alginate may be cured and lyophilized to prepare a first layer while the second alginate is cured and lyophilized to separately prepare a second layer so that the resulting layers can be adhered to each other to obtain a laminate.

Detailed description of the steps is the same as the above-described steps.

A material for preventing adhesion having a desired size, height (thickness) and shape can be obtained by using a vessel, a mold, a substrate, a porous membrane, a non-woven fabric, a woven fabric or the like having a desired size, height (thickness) and shape upon curing the first alginate and the second alginate.

Preferably, the material for preventing adhesion is further subjected to a sterilization treatment. Examples of sterilization include, but not limited to, gamma-ray sterilization, electron beam sterilization, ethylene oxide gas sterilization and ethanol sterilization. More preferably, the material for preventing adhesion is subjected to a sterilization treatment by electron beam and/or gamma irradiation. A polymeric material is subjected to an irradiation treatment with a gamma-ray, an electron beam or the like so as to preferably obtain a highly biocompatible medical material with controlled retention in the body (see, for example, Japanese Patent Application Publication No. 2000-237294).

Examples of the irradiation conditions upon electron beam and/or gamma-ray sterilization include an absorbed dose of 10 kGy-150 kGy, more preferably 20 kGy-100 kGy, and still more preferably 40 kGy-80 kGy. In another preferable aspect, examples of the irradiation conditions upon electron beam and/or gamma-ray sterilization include an absorbed dose of 20 kGy-80 kGy, 20 kGy-60 kGy or 40 kGy-60 kGy. Electron beam sterilization is more preferable than gamma-ray sterilization.

For example, as a sponge-like laminate before being pressed, when the shape of a plate is expressed by length × width × height (thickness), the length and the width of the material for preventing adhesion produced as such are not particularly limited while the height (thickness) is preferably 0.2 mm-30 mm, more preferably 0.3 mm-15 mm, and still more preferably 0.5 mm-10 mm. Yet still more preferably, in addition to such a height (thickness), the length and the width are 1 mm-300 mm × 1 mm-300 mm, particularly preferably 3 mm-200 mm × 3 mm-200 mm, and more preferably 5 mm-150 mm × 5 mm-150 mm, respectively.

A step of pressing a laminate obtained by the step (5) is further included. Pressing can be performed by holding and applying pressure on the laminate manually or with a press machine. Generally employed steps such as compression and thinning are also included as pressing of the present invention. Examples of the pressure adopted for pressing include 1 kPa-100 MPa, more preferably 10 kPa-80 MPa, and still more preferably 100 kPa-60 Mpa. Manual pressing is performed with a means that can apply uniform pressure onto the laminate by pressing it with a hand, for example, an acrylic ruler, an acrylic plate, a glass plate, a metal plate or the like. Moreover, an example of the press machine used includes a hot press machine (AH-1T from AS ONE Corporation).

### 6. Usage

The material for preventing adhesion is used by applying it to a subject in need of adhesion prevention. Preferably, the material for preventing adhesion remains on the applied site usually for about a week that is necessary for exhibiting the adhesion preventing effect, then absorbed and decomposed and eventually metabolized/excreted in about 1-2 months, and thus it is highly safety.

The material for preventing adhesion may also be applied to a surface of a wound, for example, a surface of a tissue involved in a surgical operation.

A "tissue involved in a surgical operation" refers to a tissue that has a wound on its surface due to the surgical operation, or a tissue that has inflammation or that has a risk of inflammation due to drying of the surface upon a surgical operation. A tissue involved in a surgical operation is preferably an organ wrapped in peritoneum (for example, stomach, jejunum, ileum, appendix, colon, liver, spleen, duodenum and pancreas). A material for preventing adhesion of a preferable aspect of the present invention is capable of effectively preventing a serious adhesion such as an adhesion occurring after hepatic resection.

To "apply" means to place a material for preventing adhesion on a surface of a wound (for example, a surface of a tissue involved in a surgical operation). Specifically, the material for preventing adhesion is placed on a surface of a wound (for example, a surface of a tissue involved in a surgical operation) such that the surface of the first layer of the material for preventing adhesion makes contact with the surface of the wound (for example, the surface of the tissue) while the surface of the second layer faces the opposite side (for example, the abdominal side) of the surface of the wound (for example, the surface of the tissue). For example, when the first layer and second layer of the material for preventing adhesion are produced such that the weight-average molecular weight of the first layer becomes higher than that of the second layer, since the first layer of the material for preventing adhesion has a relatively high weight-average molecular weight, it remains on the surface of the tissue without being dissolved for a sufficient amount of time to prevent adhesion, thereby serving as a physical barrier for the wounded surface. Meanwhile, since the second layer has a relatively low weight-average molecular weight, it melts and spreads quickly to exert adhesion prevention for the uninjured surface.

Preferably, the material for preventing adhesion is highly flexible and hard to break as compared to Seprafilm (trade name). Therefore, in a preferable aspect, application of the material for preventing adhesion is not limited to the surface of the tissue to be applied and, for example, it can also be wound around an intestinal tract upon intestinal anastomosis. **In** another preferable aspect, it can easily be inserted through a pathway for putting a surgical instrument in and out upon a surgical operation using an endoscope in a subject. In yet another preferable aspect, the material for preventing adhesion can be reattached.

Preferably, the material for preventing adhesion can be applied to a wider range of targets for adhesion prevention compared to INTERCEED (trade name).

Preferably, the material for preventing adhesion is prepared in a size appropriate for the area, shape, unevenness and the like of a surface to be applied, and applied onto the surface of the tissue involved in a surgical operation for adhesion prevention. A "subject" may be human or an organism other than human, for example, a bird or a non-human mammal (for example, bovine, monkey, cat, mouse, rat, guinea pig, hamster, pig, dog, rabbit, sheep or horse).

The material for preventing adhesion preferably has a pressed sheet shape. Since the material for preventing adhesion can be made compact by having a pressed sheet shape, the material for preventing adhesion can be applied to the affected area relatively easily, for example, via a trocar or the like upon an endoscopic surgery. Thereafter, the material for preventing adhesion applied to the affected area preferably absorbs moisture present in the affected area or moisture applied to the affected area to restore the thickness.

Similar to Seprafilm (trade name) and INTERCEED (trade name), the material for preventing adhesion can preferably be used safely in a subject.

After application to the surface of the tissue involved in a surgical operation, there is usually no need of suture between the material for preventing adhesion and the surface of the tissue involved in the surgical operation, but if necessary, the material for preventing adhesion may be sutured with the tissue involved in the surgical operation.

Furthermore, a method for preventing adhesion including a step of applying a material for preventing adhesion to a subject in need of adhesion prevention is provided. Detail of the method is as described hereinbefore.

Moreover, use of a laminate for producing a material for preventing adhesion is provided. Detail of the use is as described hereinbefore.

In addition, a laminate for preventing adhesion is provided. Detail of the laminate is as described hereinbefore.

In addition, an alginate for being used in adhesion prevention, by which a material for preventing adhesion is applied to a subject wound is provided. Detail of the alginate is as described hereinbefore.

### 7. Co-administered drug

Moreover, a co-administered drug, for example, an antibiotic such as streptomycin, penicillin, tobramycin, amikacin, gentamicin, neomycin or amphotericin B or an anti-inflammation drug such as aspirin, a non-steroidal analgesic antipyretic drug (NSAIDs) or acetaminophen may be administered before, simultaneously or after applying the material for preventing adhesion of the present invention to a tissue involved in a surgical operation. These drugs may also be used by being mixed with the material for preventing adhesion of the present invention.

Since the material for preventing adhesion is porous and has a water absorbing property, it is more adoptable for carrying a drug that can be prepared upon use, for example, as compared to non-porous Seprafilm (trade name). The sponge-like material for preventing adhesion may be impregnated with a drug solution for administration so that adhesion prevention and topical sustained release of the drug can be realized at the same time upon administration in abdominal cavity, thoracic cavity, cardiac cavity or the like. Moreover, a drug can be carried in layers with different dissolution rates so as to allow sustained release of the drug at a faster sustained release rate and a slower sustained release rate.

The present invention will be further described in detail by way of examples, although the present invention should not be limited to these examples.

### Examples

### Example 1: Production of alginic acid sheet

An alginic acid-layered sponge-like composition (alginic acid sheet) of Example 1, which was to be used in each experiment example described below, was produced as follows.

### [Reagents]

The reagents used for producing the alginic acid sheet were as follows.

Low-endotoxin sodium alginate was obtained from Mochida Pharmaceutical Co., Ltd.
· AL10: (Lot NO. 8B19202), endotoxin level 2 EU/g.
· AL500: (Lot NO. 9J02121), endotoxin level 12 EU/g.

Calcium chloride was obtained from Wako Pure Chemical Industries, Ltd. (Product code: 036-00485).

Otsuka distilled water was obtained from Otsuka Pharmaceutical Factory, Inc. (Product code: 035206903).

### [Instruments used]

Resin square tray (inside dimensions: W65 mm × D65 mm × H10 mm)
Automatic pipetting device (Musashi Engineering, Inc.)
Vacuum lyophilizer (Model: DFM-10C-04, ULVAC, Inc.)

### [Preparation procedure]

### (1) Preparation of solution

AL500 was dissolved in Otsuka distilled water such that the concentration reached 0.33% to prepare an AL500 solution. Similarly, AL10 was dissolved in Otsuka distilled water such that the concentration reached 1% to prepare an AL10 solution. Furthermore, calcium chloride was dissolved in Otsuka distilled water to prepare 4.0 mM and 9.6 mM calcium chloride aqueous solutions, respectively.

### (2) Preparation of AL500 layer (lower layer)

7.0 mL of the AL500 solution and 7.0 mL of the 4.0 mM calcium chloride aqueous solution were added while being uniformly mixed to the resin square tray with the automatic pipetting device.

### (3) Lamination of AL10 layer (upper layer)

7.0 mL of the AL10 solution and 7.0 mL of the 9.6 mM calcium chloride aqueous solution were added while being uniformly mixed onto an AL500 layer (lower layer) produced in (2) with the automatic pipetting device.

### (4) Production of sponge-like composition

The resin square tray including two layers produced in (3) was set in the lyophilizer and lyophilized by a common method, thereby obtaining an alginic acid-layered sponge-like composition of interest.

The alginic acid-layered sponge-like composition of interest included the sponge-like lower layer (that is, a first layer) containing AL500 and calcium chloride, and the sponge-like upper layer containing AL10 and calcium chloride (that is, a second layer). The alginic acid-layered sponge-like composition was a square that was about 65 mm in length for each side thereof and about 3 mm in thickness. The total amount of sodium alginate used in the upper layer and the lower layer was about 93 mg/composition (the amount per 72 cm² disclosed in Table 1 below reached about 160 mg). The ratio (weight ratio) of the sodium alginate used in the upper layer and the lower layer was 3: 1. The total amount of calcium chloride used in the upper layer and the lower layer was about 10 mg/composition (the amount per 72 cm² disclosed in Table 1 below reached about 18 mg).

### (5) Measurement of weight-average molecular weight

The weight-average molecular weight of the sodium alginate used as a production feedstock was measured by GPC-MALS method below.

### [Pretreatment method]

An eluent was added to dissolve a specimen, which was filtrated through a 0.45 µm membrane filter to obtain a measurement solution.

### [Measurement conditions (refractive index increment (dn/dc) determination)]

Differential refractometer: Optilab T-rEX
Measurement wavelength: 658 nm
Measurement temperature: 40°C
Solvent: 200 mM aqueous sodium nitrate solution
Specimen concentration: 0.5-2.5 mg/mL (5 concentrations)

### [Measurement conditions (absolute molecular weight distribution determination)]

Columns: TSK gel GMPW-XL × 2 + G2500PW-XL (7.8 mm I.D. × 300 mm × 3 columns)
Eluent: 200 mM aqueous sodium nitrate solution
Flow rate: 1.0 mL/min.
Concentration: 0.05%
Detector: RI detector, light scattering detector (MALS)
Column temperature: 40°C
Injection amount: 200 µL

### [Results]

AL10: 55,000
AL500: 280,000

A single-layer sponge-like composition containing AL10 and a single-layer sponge-like composition containing AL500 prepared according to the procedures of steps (1), (2), and (4) above were subjected to electron beam sterilization and then dissolved in an EDTA (ethylenediaminetetraacetic acid) solution to respectively measure their molecular weights by GPC-MALS method. The results are shown below.

### [Results]

(Where the radiation dose for electron beam sterilization was 20 kGy)
   AL10: 36,000
   AL500: 75,000
(Where the radiation dose for electron beam sterilization was 40 kGy)
   AL10: 27,000
   AL500: 45,000

### (6) Production of alginic acid sheet by pressing of sponge-like composition

The sponge-like composition obtained in (4) above was set in a press machine (from AS ONE Corporation, product name AH-1T). The sponge-like composition was pressed at a pressure of 10 MPa at room temperature and held for 5 minutes. The pressed sponge-like composition (alginic acid sheet) was sterilized with an electron beam according to a common method. The resulting alginic acid sheet was used as Example 1 in each experiment example described below.

The preparation of the alginic acid sheet according to each of examples and comparative examples including Example 1 is shown in Table 1 together with the results and the like of Experiment Example 2-2.

### Examples 2 to 6: Production of alginic acid sheets

Alginic acid sheets of Examples 2 to 6 were produced by the same method as in the production of the alginic acid sheet of Example 1 except that the total amount of the amounts of calcium chloride used and the ratio of the amounts of calcium chloride used in the upper layer to the lower layer were changed to preparations in Table 1. The produced alginic acid sheet of each example was used in each experiment example described below.

### Examples 7 to 9: Production of alginic acid sheets

Alginic acid sheets of Examples 7 to 9 were produced by the same method as in the production of the alginic acid sheet of Example 1 except that, with AL10 that is used in Example 1 being used as alginic acid used in an upper layer and AL500 that is used in Example 1 being used as alginic acid used in a lower layer, the total amount of the amounts of sodium alginate used in the upper layer and the lower layer and the ratio of the amounts of sodium alginate used in the upper layer to the lower layer were changed to preparations in Table 1, and the total amount of the amounts of calcium chloride used and the ratio of the amounts of calcium chloride used in the upper layer to the lower layer were changed to preparations in Table 1. The produced alginic acid sheet of each example was used in each experiment example described below.

### Examples 10 and 11: Production of alginic acid sheets

Alginic acid sheets of Examples 10 and 11 were produced by the same method as in the production of the alginic acid sheet of Example 1 except that AL500 used in Example 1 was used as alginic acid used in an upper layer (that is, AL500 was used in both the upper layer and a lower layer), the total amount of the amounts of sodium alginate used in the upper layer and the lower layer and the ratio of the amounts of sodium alginate used in the upper layer to the lower layer were changed to preparations in Table 1, and the total amount of the amounts of calcium chloride used and the ratio of the amounts of calcium chloride used in the upper layer to the lower layer were changed to preparations in Table 1. The produced alginic acid sheet of each example was used in each experiment example described below.

### Examples 12 and 13: Production of alginic acid sheets

AL10 used in Example 1 and AL500 used in Example 1 were used as alginic acid, AL10 solutions and AL500 solutions were prepared according to the method of Example 1 in accordance with preparations in Table 1, and both were uniformly mixed. In addition, a calcium chloride aqueous solution was prepared according to the method of Example 1. The total amount of the amounts of sodium alginate used and the total amount of the amounts of calcium chloride used were set according to preparations in Table 1, and alginic acid sheets (single layer) of Example 12 and Example 13 were produced according to the method with [preparation procedures] of (1), (2), and (4) in the production of the alginic acid sheet of Example 1. The produced alginic acid sheet of each example was used in each experiment example described below.

### Comparative Examples 1 to 4: Production of alginic acid sheets

Alginic acid sheets of Comparative Examples 1 to 4 were produced by the same method as in the production of the alginic acid sheet of Example 1 except that AL10 or AL500 used in Example 1 was used as alginic acid used in an upper layer and alginic acid used in a lower layer according to combinations shown in Table 1, the total amount of the amounts of sodium alginate used in the upper layer and the lower layer and the ratio of the amounts of sodium alginate used in the upper layer to the lower layer were changed to preparations in Table 1, and the total amount of the amounts of calcium chloride used and the ratio of the amounts of calcium chloride used in the upper layer to the lower layer were changed to preparations in Table 1. The produced alginic acid sheet of each comparative example was used in each experiment example described below.

### Comparative Examples 5 and 6: Production of alginic acid sheets

An alginic acid sheet of Comparative Example 5 was produced by the same method as in the production of the alginic acid sheet of Example 1 except that, in a combination shown in Table 1, the total amount of the amounts of calcium chloride used and the ratio of the amounts of calcium chloride used in the upper layer to the lower layer were changed to a preparation in Table 1. In addition, an alginic acid sheet of Comparative Example 6 was produced by the same method as in the production of the alginic acid sheet of Example 12 except that the total amount of the amounts of calcium chloride used was changed to a preparation in Table 1. The produced alginic acid sheet of each comparative example was used in each experiment example described below.

### Experiment Example 1-1: Dissolution test of alginic acid sheet (production of agar)

Agar used in Experiment Example 1-2 described below was produced. Detail of the procedure will be described below.

### [Materials]

· Agar (powder) (manufactured by Wako Pure Chemical Industries, Ltd., Product code: 010-0875)
· Pure water
· Physiological saline solution (manufactured by Hikari Pharmaceutical Co., Ltd.)

### [Instruments used]

· Microwave (MC-E2 manufactured by NEC Corporation)
· Tray dedicated for agar production (Gel Casting Set-L, Mupid Co., Ltd.)

### [Procedure]

First, 200 mL of pure water and 4 g of an agar powder were put into a 300 mL beaker and heated with the microwave for 2-3 minutes in a manner of preventing bumping to dissolve the agar (powder). Next, the agar (powder) was stirred with a stirrer, and the solution was made to flow into the tray dedicated for agar production. Then, the solution was left to stand at room temperature until the temperature decreased and the agar solidified. After that, the produced agar was immersed in the physiological saline solution for one night or longer, and the physiological saline solution was infiltrated into the agar.

### Experiment Example 1-2: Dissolution test of alginic acid sheet (semi-immersion test)

Dissolution tests were performed using the alginic acid sheets produced as Examples 1 to 13 and Comparative Examples 1 to 6. Detail of the procedure will be described below.

### [Materials]

· Agar produced in Experiment Example 1-1
· Alginic acid sheets produced as Examples 1 to 13 and Comparative Examples 1 to 6
· Physiological saline solution (manufactured by Hikari Pharmaceutical Co., Ltd.)

### [Instruments used]

· 8 mm-diameter biopsy punch (BP-80F (trade name), Kai medical)
· 10 cm-diameter dish (IWAKI, Product code: 3020-100)
· 100 µm cell strainer (FALCON)
· Electronic balance (Shimadzu Corporation, AUW220D (trade name))
· Shaker (TAITEC, Triple shaker NR-80 (trade name))

### [Procedure]

First, as advance preparation, the side surface of the cell strainer was cut off to use the bottom surface of the cell strainer as a mesh, and the weight was measured. In addition, the alginic acid sheets produced as Examples 1 to 13 and Comparative Examples 1 to 6 were hollowed out with the biopsy punch, and the weights were measured. Furthermore, the agar produced in Experiment Example 2-1 was cut into 1 cm × 1 cm (thickness: 0.7 cm) sizes.

Next, the cut agar was put into the 10 cm-diameter dish, and 28 mL of the physiological saline solution was poured into the dish. The mesh on which a sample of each alginic acid sheet was placed was placed on the agar, and the 10 cm dish was placed on the shaker and started to be shaken at room temperature at 40 shakes/min. and an amplitude of 25 cm.

After that, the mesh was taken out from the 10 cm dish every certain time, moisture was removed by bringing a dry paper waste cloth (KIMWIPES (trade name), manufactured by Nippon Paper Crecia Co., Ltd.) into contact with the bottom of the mesh until no water drops on the mesh would be visually confirmed, then, the weight was measured, an appearance photo was captured as necessary, and the mesh was returned to the 10 cm dish. In addition, after the weight was measured, 200 µm of the physiological saline solution was added to the 10 cm dish. Then, the dissolution test was completed at a point in time when the sample on the mesh was completely dissolved.

### [Results]

Changes in the weights of the alginic acid sheets produced as Examples 1 to 13 and Comparative Examples 1 to 6 at each measurement time are shown in Fig. 2 to Fig. 20. As shown in Fig. 2 to Fig. 12, the samples of Examples 1 to 11 all satisfied the conditions (1) and (2). In addition, as shown in Figs. 13 and 14, Examples 12 and 13 having a single-layer structure also satisfied the conditions (1) and (2).

The samples of Comparative Examples 5 and 6 where the total amount of the amounts of calcium chloride used was further increased than that in Comparative Example 1 failed to satisfy the condition (1) in the dissolution test of Experiment Example 1-2. Specifically, for the samples, the times required for the weight of the sample to be less than 0.01 g exceeded 36 hours from the start of the test.

The samples where the total amount of the amounts of calcium chloride used was further increased than the amounts shown in Table 1 failed to satisfy the conditions (1) and (2) in the dissolution test of Experiment Example 1-2. Specifically, for the samples, the times required for the weight of the sample to be less than 0.01 g exceeded 36 hours from the start of the test, and the time required for the weight of the sample to reach the maximum weight exceeded 10 hours from the start of the test.

**[Table 1]**

| [Table 1] | Ca | | | | Alg | | | | Alg/Ca ratio | Press thickness (µm) | Solubility (1) | Solubility (2) |
|---|---|---|---|---|---|---|---|---|---|---|---|---|
| | Upper layer (mg) | Lower layer (mg) | Total amount (mg)/72 cm² | Upper layer/lower layer ratio | Upper layer (mg) | Lower layer (mg) | Total amount (mg)/72 cm² | Upper layer/lower layer ratio | | | | |
| Example 1 | 5.3 | 12.7 | 18 | 0.4 | AL10 120 mg | AL500 40 mg | 160 | 3 | 8.9 | 300 | ○ | ○ |
| Example 2 | 1.8 | 12.7 | 14.5 | 0.1 | AL10 120 mg | AL500 40 mg | 160 | 3 | 11 | 300 | ○ | ○ |
| Example 3 | 9 | 9 | 18 | 1 | AL10 120 mg | AL500 40 mg | 160 | 3 | 8.9 | 300 | ○ | ○ |
| Example 4 | 3.5 | 14.5 | 18 | 0.2 | AL10 120 mg | AL500 40 mg | 160 | 3 | 8.9 | 300 | ○ | ○ |
| Example 5 | 1.7 | 14.5 | 16.2 | 0.1 | AL10 120 mg | AL500 40 mg | 160 | 3 | 9.9 | 300 | ○ | ○ |
| Example 6 | 5.3 | 9 | 14.3 | 0.6 | AL10 120 mg | AL500 40 mg | 160 | 3 | 11 | 300 | ○ | ○ |
| Example 7 | 5.3 | 12.7 | 18 | 0.4 | AL10 120 mg | AL500 60 mg | 180 | 2 | 10 | 300 | ○ | ○ |
| Example 8 | 3.5 | 9 | 12.5 | 0.4 | AL10 80 mg | AL500 80 mg | 160 | 1 | 13 | 300 | ○ | ○ |
| Example 9 | 9 | 9 | 18 | 1 | AL10 80 mg | AL500 32 mg | 112 | 2.5 | 6.2 | 100 | ○ | ○ |
| Example 10 | 3 | 13.5 | 16.5 | 0.2 | AL500 100 mg | AL500 60 mg | 160 | 1.7 | 9.7 | 300 | ○ | ○ |
| Example 11 | 1 | 9 | 10 | 0.1 | AL500 80 mg | AL500 80 mg | 160 | 1 | 16 | 300 | ○ | ○ |
| Example 12 | - | - | 18 | - | AL10 120 mg + AL500 40 mg | | 160 | - | 8.9 | 300 | ○ | ○ |
| Example 13 | - | - | 12.5 | - | AL10 120 mg + AL500 40 mg | | 160 | - | 13 | 300 | ○ | ○ |
| Comparative Example 1 | 13 | 9 | 22 | 1.4 | AL10 120 mg | AL500 40 mg | 160 | 3 | 7.3 | 300 | ○ | ○ |
| Comparative Example 2 | 1 | 3 | 4 | 0.3 | AL10 120 mg | AL500 40 mg | 160 | 3 | 40 | 300 | × | ○ |
| Comparative Example 3 | 9 | 9 | 18 | 1 | AL10 80 mg | AL10 240 mg | 320 | 0.3 | 18 | 10 | × | ○ |
| Comparative Example 4 | 9 | 9 | 18 | 1 | AL10 40 mg | AL500 40 mg | 80 | 1 | 4.4 | 10 | × | ○ |
| Comparative Example 5 | 70 | 30 | 100 | 2.3 | AL10 120 mg | AL500 40 mg | 160 | 3 | 1.6 | 300 | × | ○ |
| Comparative Example 6 | - | - | 100 | - | AL10 120 mg + AL500 40 mg | | 160 | - | 1.6 | 300 | × | ○ |

In each of the examples and the comparative examples shown in Table 1, the Ca content converted to the weight of calcium chloride and the alginic acid content converted to the weight of sodium alginate are the content contained in 72 cm² of the material for preventing adhesion, and the size of the material for preventing adhesion can be suitably adjusted by changing the amount of the reagent and the size of the tray.

### Experimental Example 2: Partially resected rat hepatic model

Partially resected rat hepatic models were used to evaluate formation of adhesions. A partially resected rat hepatic model is a model that generates serious inflammation and that allows highly reproducible observation of highly intense adhesion formation (Shimizu A et al., (2014) Surg Today. (44): 314-323). Specifically, formation of an adhesion was evaluated as follows.

### [Materials]

Samples of Examples 1 to 3, 6 to 11, and Comparative Examples 5 and 6 were prepared as described above.

A sample of Comparative Example A was prepared by the same method as in Comparative Examples 1 to 4. As a result of a dissolution test, in the sample of the Comparative Example A, the time required for the weight of the sample to be less than 0.01 g was 52-55 hours, and the condition (1) was not satisfied.

Seprafilm (trade name) that was to be used as a positive control group was prepared. Seprafilm was a sheet-like material of a mixture of carboxymethyl cellulose (CMC) and hyaluronic acid, which was obtained from Genzyme GmbH.

### [Experimental groups]

Control group (n = 8): A model of adhesion after hepatic resection was made by resecting a piece that was about 2 cm and about 3 cm in width from the front end of the left lobe of a liver and a piece that was about 1 cm and about 2 cm in width from the front end of the middle lobe of the liver (inside right lobe: the right side of segmented lobes), respectively, using forceps and a bipolar electrosurgical unit (untreated control group).

Positive control group (n = 8): 2 × 2 cm Seprafilm was applied as a material for preventing adhesion.

Examples 1 to 3 and 6 to 11 groups (n = 1-8): A 2 × 2 cm sample of each example was applied as a material for preventing adhesion.

Comparative Examples 5 and 6 groups (n = 1-8): A 2 × 2 cm sample of each comparative examples was applied as a material for preventing adhesion.

Comparative Example A group: A sample of Comparative Example A was applied as a material for preventing adhesion.

### [Procedure]

A rat was placed under general anesthesia by administering a dose of 2.5 mL/kg of three types of mixed anesthesia into the subcutaneous tissue of the back. The abdomen was opened about 5 cm by midline abdominal incision to pull out the liver, and a model of adhesion after hepatic resection was made by resecting a piece that was about 2 cm and about 3 cm in width from the front end of the left lobe of the liver and a piece that was about 1 cm and about 2 cm in width from the front end of the middle lobe of the liver (inside right lobe: the right side of segmented lobes), respectively, using forceps and a bipolar electrosurgical unit. At the time of holding the liver, ring tweezers were used. For the control group, the abdomen was closed immediately thereafter to complete the treatment. For the groups to be applied with the material for preventing adhesion, the material for preventing adhesion was applied to the resected surface of the left lobe. After the liver was returned to the original position, sutures were made in two steps in the abdominal wall and the skin to close the abdomen. The abdominal wall was sutured using a biodegradable suture while the skin was sutured with a nonabsorbable suture. A week following the abdominal closure, the rat was placed under general anesthesia by administering a dose of 2.5 mL/kg of three types of mixed anesthesia into the subcutaneous tissue of the back and then euthanized by cutting the carotid artery to cause bleeding. Thereafter, the abdomen was reopened to evaluate adhesions as follows.

### [Evaluation of adhesions]

The adhesions were evaluated as follows.

### (1) Resected surface

The following evaluation was performed on the resected surface of the left lobe of the liver described in [Procedure] above.

### Adhesion extent

The width of an adhesion formed on the 3 cm-wide resected surface of the left lobe of the liver was measured with a ruler and expressed as a length (unit: mm) (thus, the maximum extent of the resected surface would be 30 mm).

### (2) Unresected surface

The following evaluation was performed on parts other than the resected liver surface, specifically, liver surface, greater omentum, peritoneum, small intestine, a part directly under the midline wound and the like. Adhesions on the unresected surface serve as an indicator of de novo adhesions.

### Adhesion extent

The width of a tissue site with an adhesion in a site other than the resected liver surface was measured with a ruler and expressed as a length (unit: mm). The site of the adhesion was not specified, and the maximum width observed with adhesion formation was recorded as the adhesion extent of the test animal.

### [Results]

The results from the adhesion evaluations are shown in Fig. 21 regarding both the resected surface and the unresected surface.

For each example group, an adhesion on the resected surface was found to be the same as those for the control group and the positive control group or be suppressed as compared to the control group and the positive control group. In addition, for each example group, an adhesion was suppressed as compared to the Comparative Example A group and the Comparative Examples 5 and 6 groups.

A remarkable adhesion preventing effect was confirmed on the unresected surface of each example group as compared to the control group, the Comparative Example A group and the Comparative Examples 5 and 6 groups. In addition, for each example group, an adhesion was found to be the same as that for the positive control group or be suppressed as compared to the positive control group.

### Experiment Example 3: Mini pig spleen/abdominal wall adhesion model

Mini pig abdominal wall defect-induced adhesion models were used to evaluate the formation of adhesion of the material for preventing adhesion. A mini pig is an experimental animal that is considered to have a similar abdominal cavity state to that of a human being, and the abdominal wall defect-induced adhesion model is a model that allows highly reproducible observation of adhesion formation. Specifically, formation of an adhesion was evaluated as follows.

### [Materials]

Samples of Examples 1, 4, and Comparative Example 1 were prepared as described above.

Seprafilm (trade name) that was to be used as a positive control group was obtained from Genzyme GmbH.

### [Experimental groups]

Control group (n = 6): The same treatment as the following procedure was performed, the abdomen was closed without applying the material for preventing adhesion, and a control group was made (untreated control group).

Positive control group (n = 4): Three pieces of 6 × 6 cm Seprafilm were applied as a material for preventing adhesion.

Examples 1 and 4 groups (n = 3-4): A sample (6 × 6 cm, three pieces) of each example was applied as a material for preventing adhesion.

Comparative Example 1 group (n = 4): A sample (6 × 6 cm, three pieces) of Comparative Example 1 was applied as a material for preventing adhesion.

### [Procedure]

Female mini pigs (purchased from Fuji Micra, Inc., weights of 25.8-31.1 kg) were grouped to each group. Anesthesia was performed by the intramuscular administration of a liquid mixture of ketamine hydrochloride (15 mg/kg, Daiichi Sankyo Propharma) and xylazine (3 mg/kg, Bayer Yakuhin, Ltd) following the premedication of atropine (0.05 mg/kg). The inhalation anesthesia of Japanese Pharmacopoeia ISOFLURANE (Mylan Inc.) was performed under artificial respiration management (number of times of respiration: 10-15 strokes/min. and tidal volume: 5-15 ml/kg/stroke) as necessary. Oxygen was used as a carrier gas, and the concentration of ISOFLURANE was maintained at 1-3% with a vaporizer. A wide area of the midline region in a surgical field was shaved with hair clippers, washed in a low-temperature bath and then disinfected with alcohol for disinfection (Wako Pure Chemical Industries, Ltd.) and isodin solution for animals (Mundipharma K.K.; 20 mg of Japanese Pharmacopoeia povidone iodine in 1 mL). After the abdomen was opened using a monopolar electrosurgical unit or the like, the spleen and the stomach were exposed and secured. The surfaces of the spleen and the stomach were physically rubbed with a dry scrub (sterilized disposable brush), the abdominal cavity side of the open part was resected from the peritoneum using a scalpel and scissors, and a separated adhesion model was made. One piece of the material for preventing adhesion was brought into close contact with each of a scratched site opposite to the peritoneum-separated site and the vicinity thereof, the fact that the material for preventing adhesion did not move was confirmed, then, the organ was returned to the original position, one piece of the material for preventing adhesion was left to stand immediately below the incision site of the peritoneum, and the peritoneum, the muscular layer, the subcutaneous tissue, and the skin were sutured.

At the seventh day from the making of the model, exsanguination was performed by cutting the axillary artery and venous under ketamine anesthesia. After the abdomen was opened, an adhesion in the abdominal cavity was confirmed, and then the model-making site and the periphery thereof were captured with a digital camera. The adhesion state at the model-making site was evaluated with macroscopic scores, the size was measured for area measurement, and the model-making site was captured with the digital camera for recording. On the model-making surfaces of the spleen and the stomach in the scratched site, the adhesion surface and the model-making site were enucleated, and then photos were captured again.

### [Evaluation of adhesions]

The adhesions were evaluated as follows.

The scorings of adhesions, the lengths of adhesions, the areas of adhesions, and the like were macroscopically observed, and the photos thereof were captured and recorded.

Scoring of adhesion 0: No adhesion occurs, and an adhesion-free state is indicated.

Scoring of adhesion 1: An adhesion occurs, but the adhesion can be separated with gravity. A state where the adhesion is separated by simply lifting the organ with a single hand (simply flipping over the organ) is indicated.

Scoring of adhesion 2: An adhesion occurs, but blunt separation is possible. A state where the adhesion is separated by pulling away both ends of the adhesion with both hands is indicated.

Scoring of adhesion 3: An adhesion occurs, and blunt separation is not possible. A state where the tissue is damaged before the adhesion is separated by pulling away both ends of the adhesion with both hands is indicated (a state where the use of a scalpel or scissors is required).

The areas of the adhesions for the individual scores were measured, and the sums of the areas of the adhesions of score 1, score 2, and score 3 were compared.

### [Results]

The results of the sums of the areas of the adhesion of score 1, score 2, and score 3 of the right and left abdominal wall defect parts are shown in Fig. 22.

As shown in Fig. 22, in all of the Example 1 group and the Example 4 group, adhesions in the sites to which the material for preventing adhesion had been applied (locals) were suppressed as compared to the control group, the positive control group, and the Comparative Example 1 group. From these results, it was confirmed that the material for preventing adhesion of the present invention exhibits an excellent adhesion preventing effect even in large animals.

In addition, the results regarding adhesions of score 3 in the entire abdominal cavity, which are strong adhesions considered as a medical issue, are shown in Fig. 23.

As shown in Fig. 23, in all of the Example 1 group and the Example 4 group, adhesions were remarkably suppressed even in the peripheral parts of the sites to which the material for preventing adhesion had been applied as compared to the control group, the positive control group, and the Comparative Example 1 group. From these results, it was confirmed that the material for preventing adhesion of the present invention exhibits an excellent adhesion preventing effect even in large animals.

### Experiment Example 4: Two-stage resected mini pig hepatic model

Two-stage resected mini pig hepatic models were used to evaluate formation of adhesions. A two-stage resected mini pig hepatic model is a model that causes serious inflammation and that allows highly reproducible observation of highly intense adhesion formation. Specifically, formation of an adhesion was evaluated as follows.

### [Materials]

Samples of Comparative Examples 3 and 4 were prepared as described above.

Seprafilm (trade name) that was to be used as a positive control group was obtained from Genzyme GmbH.

### [Experimental groups]

Control group (n = 5): The same treatment as the following procedure was performed, the abdomen was closed without applying a material for preventing adhesion, and a control group was made (untreated control group).

Positive control group (n = 5): Three pieces of 6 × 6 cm Seprafilm (for one experiment) were applied twice as a material for preventing adhesion.

Comparative Examples 3 and 4 groups (n = 5): Samples (6 × 6 cm, three pieces, for one experiment) of Comparative Examples 3 and 4 were applied twice as a material for preventing adhesion.

### [Procedure]

Male mini pigs (purchased from Fuji Micra, Inc., weights of 22-28 kg) were grouped to each group. Anesthesia was performed by the intramuscular administration of a liquid mixture of ketamine hydrochloride (15 mg/kg, Daiichi Sankyo Propharma) and xylazine (3 mg/kg, Bayer Yakuhin, Ltd) following the premedication of atropine (0.05 mg/kg). The inhalation anesthesia of Japanese Pharmacopoeia ISOFLURANE (Mylan Inc.) was performed under artificial respiration management (number of times of respiration: 10-15 strokes/min. and tidal volume: 5-15 ml/kg/stroke) as necessary. Oxygen was used as a carrier gas, and the concentration of ISOFLURANE was maintained at 1-3% with a vaporizer. A wide area of the midline region in a surgical field was shaved with hair clippers, washed in a low-temperature bath and then disinfected with alcohol for disinfection (Wako Pure Chemical Industries, Ltd.) and isodin solution for animals (Mundipharma K.K.; 20 mg of Japanese Pharmacopoeia povidone iodine in 1 mL). After the abdomen was opened using a monopolar electrosurgical unit or the like, the liver was exposed and secured. At a first term (upon first abdominal opening), the left lobe (LL) was partially resected. At a second term (upon second abdominal opening), the middle lobe (ML) was partially resected. At the first term, the left lobe was resected such that the long diameter of the resected surface of the liver became about 8 cm, and, at the second term, the middle lobe was resected such that the long diameter of the resected surface became about 10 cm. After the completion of the resection, a total of three pieces (108 cm² per pig) of the material for preventing adhesion, that is, one piece (6 × 6 cm) of the material for preventing adhesion for each of the LL surface, the ML surface, and the hepatic portal region, were attached, and the peritoneum, the muscular layer, the subcutaneous tissue, and the skin were sutured.

The resection of the hepatic portal region and the attachment of the material for preventing adhesion at the second term were performed in the same manner as described above after two weeks from the first term.

At the 28th day from the making of the model, exsanguination was performed by cutting the axillary artery and venous under ketamine anesthesia. After the abdomen was opened, an adhesion in the abdominal cavity was confirmed, and then the model-making site and the periphery thereof were captured with a digital camera. The adhesion state at the model-making site was evaluated with macroscopic scores, the size was measured for area measurement, and the model-making site was captured with the digital camera for recording.

### [Evaluation of adhesions]

Adhesions were evaluated in the same manner as in Experiment Example 3.

### [Results]

The results from the adhesion evaluations are shown in Fig. 24.

As shown in Fig. 24, in the Comparative Example 3 group and the Comparative Example 4 group, adhesions were slightly suppressed as compared to the control group, but the areas of the adhesions were wider than that in the positive control group. From these results, it was confirmed that materials for preventing adhesion that do not satisfy (1) and/or (2) in the dissolution test have a low adhesion preventing effect in large animals.

### Experiment Example 5: Partially resected rat hepatic model

According to Experiment Example 2, partially resected rat hepatic models were used to evaluate formation of adhesions.

### [Materials]

Samples of Example 1 and Example 12 were prepared as described above.

Seprafilm (trade name) that was to be used as a positive control group was prepared as described above.

### [Experimental groups]

Control group (n = 8): A model of adhesion after hepatic resection was made by resecting a piece that was about 2 cm and about 3 cm in width from the front end of the left lobe of a liver using forceps and a bipolar electrosurgical unit (untreated control group).

Positive control group (n = 8): 2 × 2 cm Seprafilm was applied as a material for preventing adhesion.

Example 1 and Example 12 groups (n = 8 for each): A 2 × 2 cm sample of each example was applied as a material for preventing adhesion.
Example 1(A): Attached to the resected surface
Example 1(B): Attached to the vicinity of the resected surface
Example 12(A): Attached to the resected surface
Example 12(B): Attached to the vicinity of the resected surface

### [Procedure]

A rat was placed under general anesthesia by administering a dose of 2.5 mL/kg of three types of mixed anesthesia into the subcutaneous tissue of the back. The abdomen was opened about 5 cm by midline abdominal incision to pull out the liver, and a model of adhesion after hepatic resection was made by resecting a piece that was about 2 cm and about 3 cm in width from the front end of the left lobe of the liver using forceps and a bipolar electrosurgical unit. At the time of holding the liver, ring tweezers were used. For the control group, the abdomen was closed immediately thereafter to complete the treatment. For the groups to be applied with the material for preventing adhesion, the material for preventing adhesion was applied to the resected surface of the left lobe (positive control Example 1(A), and Example 12(A)) or to the vicinity of the resected surface of the left lobe (Example 1(B) and Example 12(B)). After the liver was returned to the original position, sutures were made in two steps in the abdominal wall and the skin to close the abdomen. The abdominal wall was sutured using a biodegradable suture while the skin was sutured with a nonabsorbable suture. A week following the abdominal closure, the rat was placed under general anesthesia by administering a dose of 2.5 mL/kg of three types of mixed anesthesia into the subcutaneous tissue of the back and then euthanized by cutting the carotid artery to cause bleeding. Thereafter, the abdomen was reopened to evaluate adhesions as follows.

### [Evaluation of adhesions]

The adhesions were evaluated as follows.

### (1) Resected surface (adhesion strength by adhesion grade)

The adhesion was evaluated by visual observation. The adhesion at a part other than the resected liver surface was scored based on the following scoring method. The part with the adhesion was not specified, and the maximum adhesion score observed was recorded as the adhesion score of the test animal.

### Scoring of adhesion:

Grade 0: No adhesion is observed
Grade 1: An adhesion that can be separated with gravity (physiological adhesion)
Grade 2: An adhesion that can be separated with tweezers (blunt adhesion)
Grade 3: An adhesion that cannot be separated without scissors or a scalpel (sharp adhesion)

### (2) Resected surface (Extent)

The following evaluation was performed on the resected surface of the left lobe of the liver described in [Procedure] above.

### Adhesion extent

The width of an adhesion formed on the 3 cm-wide resected surface of the left lobe of the liver was measured with a ruler and expressed as a length (unit: mm) (thus, the maximum extent of the resected surface would be 30 mm).

### [Results]

The results from the adhesion evaluations are shown in Fig. 25 regarding the adhesion strength and in Fig. 26 regarding the extent.

As shown in Fig. 25, while adhesions of Grade 3, which are strong adhesions considered as a medical issue, were confirmed in all examples for the control group, for Example 1(B) and Example 12(A), adhesions of Grade 2 occurred in one of eight examples and a decrease in the adhesion strength was confirmed. In Example 1(A), a particularly remarkable effect was found, Grade 0 (no adhesions are shown) occurred in three of eight examples, and a remarkable decrease in the adhesion strength was confirmed.

As shown in Fig. 26, in Example 1(A) and Example 12(A) where the sample was attached to the resected liver surface, a remarkable decrease in the length of the adhesion on the resected liver surface was found, and an excellent adhesion preventing effect was confirmed. **In** addition, in Example 1(B) and Example 12(B) where the sample was attached not to the resected liver surface but to the vicinity thereof, as compared to the case where the sample was attached to the resected liver surface, the adhesion preventing effect slightly deteriorated, but an adhesion preventing effect about as strong as that of the positive control was confirmed to be obtained.

From the above results, the material for preventing adhesion of the present invention was confirmed to exhibit an excellent adhesion preventing effect as a material for preventing adhesion used upon the operation of liver cancer requiring multiple times of operations. In addition, in a case where bile leakage on the resected surface is concerned, an adhesion preventing effect about as strong as that of the positive control was confirmed to be obtained by attaching the material for preventing adhesion not to the resected surface but to the vicinity thereof.

Even when the test is performed using the sample of Example 13 instead of the sample of Example 12, the same results as those from the sample of Example 12 are obtained.

### [Reference Signs List]

- 1: Material for preventing adhesion
- 2: First layer
- 3: Second layer
- 4: Laminate

## Claims

1. A sheet-like material for preventing adhesion which has been pressed and contains alginate, and at least a portion of which is crosslinked with a curing agent,
the material for preventing adhesion satisfying (1) and (2) when a dissolution test is performed in which a sample cut into a substantial circle having a diameter of 8 mm is left to stand, via a mesh, on agar of a petri dish to which a physiological saline solution is added substantially to a top surface of the agar, the petri dish is shaken at an amplitude of 25 mm and 40 shakes/min, and a weight of the sample is measured at least one arbitrary point in time:
(1) a time required for the weight of the sample to be less than 0.01 g is 5-36 h from a start of the test; and
(2) a time required for the weight of the sample to reach a maximum weight is 10 h or less from the start of the test.

2. The material for preventing adhesion according to claim 1 that comprises a first layer and a second layer.

3. The material for preventing adhesion according to claim 1 or 2, wherein a total amount of the alginate is 1.4 mg/cm² or more and 2.8 mg/cm² or less in terms of a weight of sodium alginate.

4. The material for preventing adhesion according to any one of claims 1 to 3, wherein the curing agent is a calcium ion compound and a total amount of calcium is 0.14 mg/cm² or more and 0.30 mg/cm² or less in terms of a weight of calcium chloride.

5. The material for preventing adhesion according to any one of claims 1 to 4, wherein a thickness of the sheet-like material for preventing adhesion is 100 µm or more and 500 µm or less.

6. The material for preventing adhesion according to any one of claims 2 to 5, wherein a dissolution rate of the first layer is slower than that of the second layer.

7. The material for preventing adhesion according to claim 1, which is a single layer.

8. A method for producing the material for preventing adhesion according to any one of claims 1 to 7, the method comprising:
(1) a step of curing (gelating) an aqueous solution of a monovalent metal salt of alginic acid with a curing agent;
(2) optionally, a step of freezing the cured (gelated) monovalent metal salt of alginic acid;
(3) optionally, a step of curing (gelating) an aqueous solution of a monovalent metal salt of alginic acid with a curing agent on a first layer to form a second layer;
(4) optionally, a step of further repeating the step (2) and the step (3) to form a third layer; and
(5) a step of lyophilizing a resulting cured material (gel).

9. The method for producing the material for preventing adhesion according to claim 8, wherein the monovalent metal salt of the alginic acid contains at least a monovalent metal salt of alginic acid having a weight-average molecular weight of 100,000 or more.

10. A sheet like material for use in a method for preventing adhesion, the method comprising:
applying to a subject in need of adhesion prevention the sheet-like material for preventing adhesion which has been pressed and contains alginate, and at least a portion of which is crosslinked with a curing agent, the material for preventing adhesion satisfying (1) and (2) when a dissolution test is performed in which a sample cut into a substantial circle having a diameter of 8 mm is left to stand, via a mesh, on agar of a petri dish to which a physiological saline solution is added substantially to a top surface of the agar, the petri dish is shaken at an amplitude of 25 mm and 40 shakes/min, and a weight of the sample is measured at least one arbitrary point in time:
(1) a time required for the weight of the sample to be less than 0.01 g is 5-36 h from a start of the test; and
(2) a time required for the weight of the sample to reach a maximum weight is 10 h or less from the start of the test.

## Patentansprüche

1. Folienartiges Material zur Verhinderung einer Haftung, das gepresst wurde und Alginat enthält und von dem wenigstens ein Teil mit einem Härtungsmittel vernetzt ist,
wobei das Material zur Verhinderung der Haftung die Bedingungen (1) und (2) erfüllt, wenn ein Auflösungstest durchgeführt wird, bei dem eine im Wesentlichen kreisförmig ausgeschnittene Probe mit einem Durchmesser von 8 mm über ein Netz auf Agar in einer Petri-Schale stehen gelassen wird, in die eine physiologische Kochsalzlösung im Wesentlichen auf die obere Fläche des Agars gegeben wird, die Petri-Schale mit einer Amplitude von 25 mm und mit 40 Schüttelbewegungen/min geschüttelt wird und das Gewicht der Probe zu wenigstens einem beliebigen Zeitpunkt gemessen wird:
(1) die erforderliche Zeit, damit das Gewicht der Probe kleiner als 0,01 g beträgt, ist 5-36 h seit Beginn des Tests; und
(2) die erforderliche Zeit, damit das Gewicht der Probe ein Maximalgewicht erreicht, ist 10 h oder weniger seit Beginn des Tests.

2. Material zur Verhinderung einer Haftung gemäß Anspruch 1, das eine erste Schicht und eine zweite Schicht umfasst.

3. Material zur Verhinderung einer Haftung gemäß Anspruch 1 oder 2, wobei die Gesamtmenge des Alginats 1,4 mg/cm² oder mehr und 2,8 mg/cm² oder weniger beträgt, bezogen auf das Gewicht von Natriumalginats.

4. Material zur Verhinderung einer Haftung gemäß einem der Ansprüche 1 bis 3, wobei das Härtungsmittel eine Calciumionenverbindung ist und die Gesamtmenge des Calciums 0,14 mg/cm² oder mehr und 0,30 mg/cm² oder weniger beträgt, bezogen auf das Gewicht von Calciumchlorids.

5. Material zur Verhinderung einer Haftung gemäß einem der Ansprüche 1 bis 4, wobei die Dicke des folienartigen Materials zur Verhinderung einer Haftung 100 µm oder mehr und 500 µm oder weniger beträgt.

6. Material zur Verhinderung einer Haftung gemäß einem der Ansprüche 2 bis 5, wobei die Auflösungsgeschwindigkeit der ersten Schicht geringer ist als die der zweiten Schicht.

7. Material zur Verhinderung einer Haftung gemäß Anspruch 1, bei dem es sich um eine einzelne Schicht handelt.

8. Verfahren zur Herstellung des Materials zur Verhinderung einer Haftung gemäß einem der Ansprüche 1 bis 7, wobei das Verfahren umfasst:
(1) einen Schritt des Härtens (Gelierenlassens) einer wässrigen Lösung eines Salzes eines einwertigen Metalls von Alginsäure mit einem Härtungsmittel;
(2) gegebenenfalls einen Schritt des Gefrierenlassens des gehärteten (gelierten) Salzes eines einwertigen Metalls von Alginsäure des einwertigen Metalls;
(3) gegebenenfalls einen Schritt des Härtens (Gelierenlassens) einer wässrigen Lösung eines Salzes eines einwertigen Metalls von Alginsäure mit einem Härtungsmittel auf einer ersten Schicht unter Bildung einer zweiten Schicht;
(4) gegebenenfalls einen Schritt des weiteren Wiederholens von Schritt (2) und Schritt (3) unter Bildung einer dritten Schicht; und
(5) einen Schritt des Lyophilisierens eines resultierenden gehärteten Materials (Gels).

9. Verfahren zur Herstellung des Materials zur Verhinderung einer Haftung gemäß Anspruch 8, wobei das Salz eines einwertigen Metalls von Alginsäure wenigstens ein Salz eines einwertigen Metalls von Alginsäure mit einem Gewichtsmittel des Molekulargewichts von 100000 oder mehr enthält.

10. Folienartiges Material zur Verwendung in einem Verfahren zur Verhinderung einer Haftung, wobei das Verfahren umfasst:
Auftragen des folienartigen Materials zur Verhinderung einer Haftung, das gepresst wurde und Alginat enthält und von dem wenigstens ein Teil mit einem Härtungsmittel vernetzt ist, auf einen Probanden, der eine Verhinderung einer Haftung benötigt, wobei das Material zur Verhinderung der Haftung die Bedingungen (1) und (2) erfüllt, wenn ein Auflösungstest durchgeführt wird, bei dem eine im Wesentlichen kreisförmig ausgeschnittene Probe mit einem Durchmesser von 8 mm über ein Netz auf Agar in einer Petri-Schale stehen gelassen wird, in die eine physiologische Kochsalzlösung im Wesentlichen auf die obere Fläche des Agars gegeben wird, die Petri-Schale mit einer Amplitude von 25 mm und mit 40 Schüttelbewegungen/min geschüttelt wird und das Gewicht der Probe zu wenigstens einem beliebigen Zeitpunkt gemessen wird:
(1) die erforderliche Zeit, damit das Gewicht der Probe kleiner als 0,01 g beträgt, ist 5-36 h seit Beginn des Tests; und
(2) die erforderliche Zeit, damit das Gewicht der Probe ein Maximalgewicht erreicht, ist 10 h oder weniger seit Beginn des Tests.

## Revendications

1. Matériau stratifié pour empêcher l'adhérence qui a été pressé et contient de l'alginate, et dont au moins une partie est réticulée avec un agent de durcissement,
le matériau pour empêcher l'adhérence satisfaisant à (1) et (2) lorsqu'un essai de dissolution est réalisé dans lequel un échantillon découpé en un cercle substantiel ayant un diamètre de 8 mm est laissé au repos, par l'intermédiaire d'un maillage, sur une gélose d'une boîte de Pétri à laquelle une solution saline physiologique est ajoutée sensiblement sur une surface supérieure de la gélose, la boîte de Pétri est secouée à une amplitude de 25 mm et 40 agitations/min, et un poids de l'échantillon est mesuré à au moins un moment arbitraire :
(1) un temps requis pour que le poids de l'échantillon soit inférieur à 0,01 g est de 5-36 h à compté d'un début de l'essai ; et
(2) un temps nécessaire pour que le poids de l'échantillon atteigne un poids maximal est inférieur ou égal à 10 h à compter du début de l'essai.

2. Matériau pour empêcher l'adhérence selon la revendication 1 qui comprend une première couche et une deuxième couche.

3. Matériau pour empêcher l'adhérence selon la revendication 1 ou 2, dans lequel une quantité totale de l'alginate est supérieure ou égale à 1,4 mg/cm² et inférieure ou égale à 2,8 mg/cm² en poids d'alginate de sodium.

4. Matériau pour empêcher l'adhérence selon l'une quelconque des revendications 1 à 3, dans lequel l'agent de durcissement est un composé ionique de calcium et une quantité totale de calcium est supérieure ou égale à 0,14 mg/cm² et inférieure ou égale à 0,30 mg/cm² en poids de chlorure de calcium.

5. Matériau pour empêcher l'adhérence selon l'une quelconque des revendications 1 à 4, dans lequel une épaisseur du matériau stratifié pour empêcher l'adhérence est supérieure ou égale à 100 µm et inférieure ou égale à 500 µm.

6. Matériau pour empêcher l'adhérence selon l'une quelconque des revendications 2 à 5, dans lequel une vitesse de dissolution de la première couche est plus lente que celle de la deuxième couche.

7. Matériau pour empêcher l'adhérence selon la revendication 1, qui est une couche unique.

8. Procédé de production du matériau pour empêcher l'adhérence selon l'une quelconque des revendications 1 à 7, le procédé comprenant :
(1) une étape de durcissement (gélification) d'une solution aqueuse d'un sel métallique monovalent d'acide alginique avec un agent de durcissement ;
(2) éventuellement, une étape de congélation du sel métallique monovalent d'acide alginique durci (gélifié) ;
(3) éventuellement, une étape de durcissement (gélification) d'une solution aqueuse d'un sel métallique monovalent d'acide alginique avec un agent de durcissement sur une première couche pour former une deuxième couche ;
(4) éventuellement, une étape de répétition supplémentaire de l'étape (2) et de l'étape (3) pour former une troisième couche ; et
(5) une étape de lyophilisation d'un matériau durci (gel) résultant.

9. Procédé de production du matériau pour empêcher l'adhérence selon la revendication 8, dans lequel le sel métallique monovalent de l'acide alginique contient au moins un sel métallique monovalent d'acide alginique présentant une masse moléculaire moyenne en poids supérieure ou égale à 100 000.

10. Matériau stratifié pour utilisation dans un procédé pour empêcher l'adhérence, le procédé comprenant :
l'application, sur un sujet nécessitant la prévention d'adhérence, du matériau stratifié pour empêcher l'adhérence qui a été pressé et contient de l'alginate, et dont au moins une partie est réticulée avec un agent de durcissement, le matériau pour empêcher l'adhérence satisfaisant à (1) et (2) lorsqu'un essai de dissolution est réalisé dans lequel un échantillon découpé en un cercle substantiel présentant un diamètre de 8 mm est laissé au repos, par l'intermédiaire d'un maillage, sur gélose d'une boîte de Pétri à laquelle une solution saline physiologique est ajoutée sensiblement sur une surface supérieure de la gélose, la boîte de Pétri est agitée à une amplitude de 25 mm et 40 agitations/min, et un poids de l'échantillon est mesuré à au moins un moment arbitraire :
(1) un temps nécessaire pour que le poids de l'échantillon soit inférieur à 0,01 g est de 5-36 h à compté d'un début de l'essai ; et
(2) un temps nécessaire pour que le poids de l'échantillon atteigne un poids maximal est inférieur ou égal à 10 h à compter du début de l'essai.
